(19) Europäisches Patentamt
European Patent Office
Office européen des brevets

(11) Veröffentlichungsnummer: **0 302 014 A1**

# (12) EUROPÄISCHE PATENTANMELDUNG

(21) Anmeldenummer: **88810499.9**

(22) Anmeldetag: **20.07.88**

(51) Int. Cl.[4]: **C 07 D 277/36**
C 07 D 277/74,
C 07 D 263/58,
C 07 D 285/08, A 01 N 47/30,
A 01 N 47/00, C 07 D 249/12,
C 07 D 285/12

(30) Priorität: **29.07.87 CH 2910/87**
**02.06.88 CH 2092/88**

(43) Veröffentlichungstag der Anmeldung:
**01.02.89 Patentblatt 89/05**

(84) Benannte Vertragsstaaten:
**BE CH DE FR GB IT LI NL**

(71) Anmelder: **CIBA-GEIGY AG**
**Klybeckstrasse 141**
**CH-4002 Basel (CH)**

(72) Erfinder: **Ehrenfreund, Josef, Dr.**
**Amselstrasse 11**
**CH-4123 Allschwil (CH)**

**Böger, Manfred**
**Wilhelm Glockstrasse 14**
**D-7858 Weil am Rhein 5 (DE)**

**Drabek, Jozef, Dr.**
**Benkenstrasse 12**
**CH-4104 Oberwil (CH)**

## (54) Phenylthioharnstoffe, -isothioharnstoffe und -carbodiimide, ihre Herstellung und Verwendung bei der Kontrolle von Schädlingen.

(57) Neue substituierte Phenylthioharnstoffe, -isothioharnstoffe und -carbodiimide der Formel I

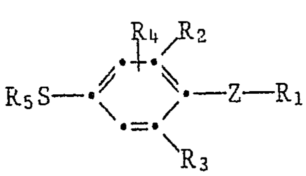

(I),

worin $R_1$ für $C_1$-$C_{10}$-Alkyl, ein- oder mehrfach durch Halogen und/oder $C_1$-$C_6$-Alkoxy substituiertes $C_1$-$C_{10}$-Alkyl, $C_3$-$C_6$-Alkenyl, $C_3$-$C_8$-Cycloalkyl, ein- oder mehrfach durch Halogen und/oder $C_1$-$C_4$-Alkyl substituiertes $C_3$-$C_8$-Cycloalkyl, $C_3$-$C_8$-Cycloalkyl-$C_1$-$C_4$-alkyl, $(C_3$-$C_8$-Cycloalkyl$)_2$-$C_1$-$C_4$-alkyl, Phenyl-$C_1$-$C_4$-alkyl, ein- oder mehrfach im Phenylteil durch Halogen, $C_1$-$C_4$-Alkyl, $C_1$-$C_4$-Alkoxy und/oder Halogen-$C_1$-$C_4$-alkyl substituiertes Phenyl-$C_1$-$C_4$-alkyl oder $C_5$-$C_6$-Cycloalkenyl;
$R_2$ für Wasserstoff, $C_1$-$C_5$-Alkyl oder $C_5$-$C_6$-Cycloalkyl;
$R_3$ für $C_1$-$C_5$-Alkyl oder $C_5$-$C_6$-Cycloalkyl;
$R_4$ für Wasserstoff oder $C_1$-$C_3$-Alkyl;
$R_5$ für einen fünfgliedrigen Heterocyclus mit 1 bis 4 gleichen oder verschiedenen Heteroatomen der Gruppe N, O und S oder einen ein- oder mehrfach durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio und/oder gegebenenfalls substituiertes Phenyl substituierten Heterocyclus mit 1 bis 4 gleichen oder verschiedenen Heteroatomen der Gruppe N, O und S;
Z für -NH-CS-NH-, $-N=C(SR_6)$-NH- oder $-N=C=N-$; und
$R_6$ für $C_1$-$C_5$-Alkyl, $C_2$-$C_5$-Alkenyl, $C_3$-$C_4$-Alkinyl, $C_1$-$C_4$-Alkoxy-$C_1$-$C_4$-alkyl oder $C_1$-$C_4$-Alkylthio-$C_1$-$C_4$-alkyl
stehen, und ihre Salze, Verfahren und Zwischenprodukte zu ihrer Herstellung, ihre Verwendung in der Schädlingsbekämpfung und Schädlingsbekämpfungsmittel, welche mindestens eine Verbindung der Formel I als Wirkstoff enthalten, werden offenbart. Bevorzugtes Anwendungsgebiet ist die Bekämpfung von Schädlingen an Tieren und Pflanzen.

EP 0 302 014 A1

## Beschreibung

### Phenylthioharnstoffe, -isothioharnstoffe und -carbodiimide, ihre Herstellung und Verwendung bei der Kontrolle von Schädlingen

Die vorliegende Erfindung betrifft neue substituierte Phenylthioharnstoffe, -isothioharnstoffe und -carbodiimide, die über eine S-Brücke mit einem fünfgliedrigen Heterocyclus verbunden sind, ihre Salze mit organischen und anorganischen Säuren, Verfahren und Zwischenprodukte zu ihrer Herstellung, Schädlingsbekämpfungsmittel, die diese Verbindungen enthalten, sowie ihre Verwendung in der Schädlingsbekämpfung.

Die erfindungsgemässen Verbindungen entsprechen der Formel I

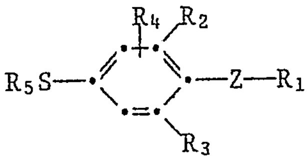

(I),

worin $R_1$ für $C_1$-$C_{10}$-Alkyl, ein- oder mehrfach durch Halogen und/oder $C_1$-$C_6$-Alkoxy substituiertes $C_1$-$C_{10}$-Alkyl, $C_3$-$C_6$-Alkenyl, $C_3$-$C_8$-Cycloalkyl, ein- oder mehrfach durch Halogen und/oder $C_1$-$C_4$-Alkyl substituiertes $C_3$-$C_8$-Cycloalkyl, $C_3$-$C_8$-Cycloalkyl-$C_1$-$C_4$-alkyl, $(C_3$-$C_8$-Cycloalkyl$)_2$-$C_1$-$C_4$-alkyl, Phenyl-$C_1$-$C_4$-alkyl, ein- oder mehrfach im Phenylteil durch Halogen, $C_1$-$C_4$-Alkyl, $C_1$-$C_4$-Alkoxy und/oder Halogen-$C_1$-$C_4$-alkyl substituiertes Phenyl-$C_1$-$C_4$-alkyl oder $C_5$-$C_6$-Cycloalkenyl;
$R_2$ für Wasserstoff, $C_1$-$C_5$-Alkyl oder $C_5$-$C_6$-Cycloalkyl;
$R_3$ für $C_1$-$C_5$-Alkyl oder $C_5$-$C_6$-Cycloalkyl;
$R_4$ für Wasserstoff oder $C_1$-$C_3$-Alkyl;
$R_5$ für einen fünfgliedrigen Heterocyclus mit 1 bis 4 gleichen oder verschiedenen Heteroatomen der Gruppe N, O und S oder einen ein- oder mehrfach durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio und/oder gegebenenfalls substituiertes Phenyl substituierten Heterocyclus mit 1 bis 4 gleichen oder verschiedenen Heteroatomen der Gruppe N, O und S;
Z für -NH-CS-NH-, -N=C($SR_6$)-NH- oder -N=C=N-; und
$R_6$ für $C_1$-$C_5$-Alkyl, $C_2$-$C_5$-Alkenyl, $C_3$-$C_4$-Alkinyl, $C_1$-$C_4$-Alkoxy-$C_1$-$C_4$-alkyl oder $C_1$-$C_4$-Alkylthio-$C_1$-$C_4$-alkyl stehen.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden Alkyle können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl usw. und ihre Isomeren genannt.

Die als Substituenten in Betracht kommenden Alkenyle können geradkettig oder verzweigt sein und eine oder mehrere Doppelbindungen aufweisen. Beispiele solcher Alkenyle sind u.a. Vinyl, Allyl, 1-Propenyl, Isopropenyl, Allenyl, Butenyle, Pentenyle oder Hexenyle.

Die als Substituenten in Betracht kommenden Alkinyle können geradkettig oder verzweigt sein. Beispiele solcher Alkinyle sind u.a. 1-Propinyl, 2-Propinyl oder die verschiedenen Butinyle.

Die als Substituenten in Betracht kommenden Phenylalkyle können geradkettig oder verzweigt sein. Geeignete Beispiele sind u.a. Benzyl, Phenäthyl, Phenpropyl, Phenisopropyl oder Phenbutyl und seine Isomeren. Ist der Phenylkern substituiert, so gelten für die Halogene, Alkyle, Alkoxide und Halogenalkyle die obigen Definitionen.

Die als Substituenten in Betracht kommenden ein- oder mehrfach durch Halogen und/oder $C_1$-$C_6$-Alkoxy substituierten $C_1$-$C_{10}$-Alkyle können geradkettig oder verzweigt und nur teilweise oder auch perhalogeniert und/oder ein- bis fünffach durch $C_1$-$C_6$-Alkoxy substituiert sein, wobei für die Halogene und die Alkyle die oben gegebenen Definitionen gelten. Geeignete Beispiele solcher Substituenten sind u.a. das ein- bis dreifach durch Fluor, Chlor und/oder Brom substituierte Methyl wie z.B. $CHF_2$ oder $CF_3$; das ein- bis fünffach durch Fluor, Chlor und/oder Brom substituierte Aethyl wie z.B. $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie z.B. $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren wie z.B. $CF(CF_3)CHFCF_3$ oder $CH_2(CF_2)_2CF_3$; Methoxymethyl, Methoxyäthyl, Aethoxyäthyl, Methoxypropyl, Aethoxypropyl, Propoxypropyl, Methoxybutyl, Aethoxybutyl, Propoxybutyl oder Butoxybutyl, 1,2-Dimethoxyäthyl, 1,3-Dimethoxypropyl oder 2,4-Dimethoxybutyl. Diese Ausführungen gelten sinngemäss auch für die gegebenenfalls ein- oder mehrfach halogenierten $C_1$-$C_4$-Alkyle.

Bei den als Substituenten in Betracht kommenden Cycloalkylen und Cycloalkenylen handelt es sich beispielsweise um Cyclopropyl, Cyclobutyl, Cyclopentyl, Cyclopentenyl, Cyclohexyl oder Cyclohexenyl. Die Cycloalkyle können ein- oder mehrfach durch einen $C_1$-$C_4$-Alkylrest substituiert und/oder über eine $C_1$-$C_4$-Alkylenbrücke mit dem Rest des Moleküls verbunden sein.

Unter den als Substituenten in Betracht kommenden fünfgliedrigen Heterocyclen seien die folgenden genannt:
$Q_1$ für Thiazolyl, $Q_2$ für 1,2,4-Triazolyl,
$Q_3$ für 1,3,4-Thiadiazolyl, $Q_4$ für 1,2,4 Thiadiazolyl,
$Q_5$ für 1,3,4-Oxadiazolyl, $Q_6$ für Thienyl,
$Q_7$ für Benzoxalyl, $Q_8$ für Benzthiazolyl,
$Q_9$ für Imidazolyl, $Q_{10}$ für Furanyl, $Q_{11}$ für Pyrrolyl,
$Q_{12}$ für Pyrazolyl, $Q_{13}$ für Oxazolyl, $Q_{14}$ für Benzofuranyl,
$Q_{15}$ für Indolyl, $Q_{16}$ für Benzimidazolyl und $Q_{17}$ für Tetrazolyl.

Als Beispiele seien u.a. die folgenden aufgeführt:

$Q_1$ = 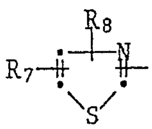 ; $Q_2$ = 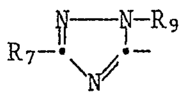 ; $Q_3$ = 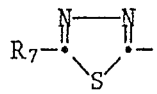 ;

$Q_4$ = 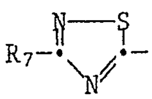 ; $Q_5$ = 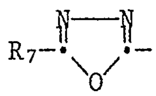 ; $Q_6$ = 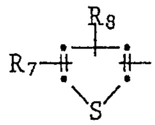

$Q_7$ = 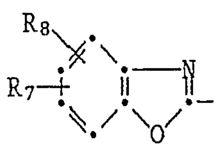 ; $Q_8$ = 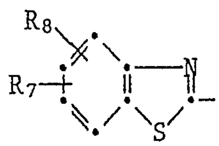 ; $Q_9$ = 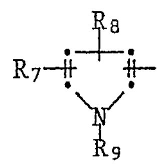 ;

$Q_{10}$ = 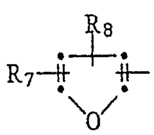 ; $Q_{11}$ = 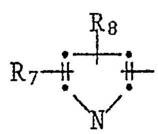 ; $Q_{12}$ = 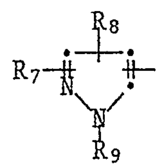 ;

$Q_{13}$ = 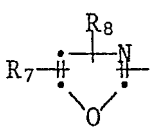 ; $Q_{14}$ = 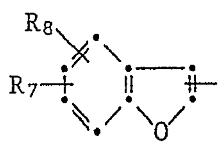 ; $Q_{15}$ = 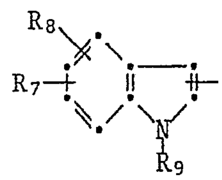 ;

$Q_{16}$ = 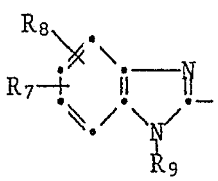 ; oder $Q_{17}$ = 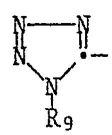 ,

wobei $R_7$ und $R_8$ je für Wasserstoff, Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio oder gegebenenfalls ein- oder mehrfach durch Halogen, $C_1$-$C_4$-Alkyl, $C_1$-$C_4$-Alkoxy und/oder Halogen-$C_1$-$C_4$-alkyl substituiertes Phenyl; and $R_9$ für Wasserstoff oder $C_1$-$C_4$-Alkyl stehen.

Die Verbindungen der Formel I, in denen Z für -N=C($SR_6$)-NH- steht, können auch in Form von Säureadditionssalzen vorliegen. Zur Bildung solcher Salze eignen sich sowohl organische als auch anorganische Säuren. Beispiele solcher Säuren sind u.a. Chlorwasserstoffsäure, Bromwasserstoffsäure, Jodwasserstoffsäure, Salpetersäure, verschiedene Phosphorsäuren, Schwefelsäure, Essigsäure, Propionsäure, Buttersäure, Valeriansäure, Oxalsäure, Malonsäure, Bernsteinsäure, Aepfelsäure, Maleinsäure, Fumarsäure, Milchsäure, Weinsäure, Zitronensäure, Benzoesäure, Phthalsäure, Zimtsäure, Phenylsulfonsäuren oder Salicylsäure.

Verbindungen der Formel I, in denen Z für -N = C($SR_6$)-NH- steht, können in den tautomeren Formen

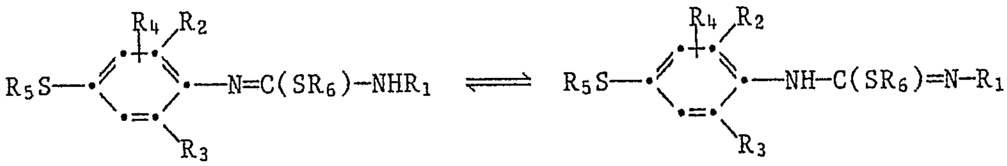

vorliegen. Die Erfindung umfasst sowohl die einzelnen Tautomeren, als auch Tautomerengemische.

Unter den Verbindungen der Formel I stehen diejenigen im Vordergrund, in denen $R_1$ für $C_1$-$C_7$-Alkyl, ein- oder mehrfach durch Halogen und/oder $C_1$-$C_5$-Alkoxy substituiertes $C_1$-$C_7$-Alkyl, $C_5$-$C_6$-Cycloalkyl, $C_3$-$C_5$-Cycloalkyl-$C_1$-$C_3$-alkyl oder ($C_3$-$C_5$-Cycloalkyl)$_2$-$C_1$-$C_3$-alkyl; $R_2$ für $C_1$-$C_4$-Alkyl oder $C_5$-$C_6$-Cycloalkyl; $R_3$ für $C_1$-$C_4$-Alkyl; $R_4$ für Wasserstoff; $R_5$ für

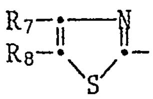 , 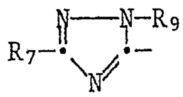 , 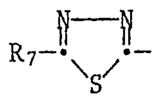 oder 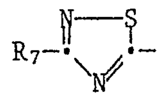 ,

wobei $R_7$ und $R_8$ Wasserstoff, Chlor, Brom, $C_1$-$C_4$-Alkyl oder Halogen-$C_1$-$C_4$-alkyl und $R_9$ $C_1$-$C_5$-Alkyl bedeuten; Z für -NH-CS-NH-, -N = C($SR_6$)-NH- oder -N = C = N-; und $R_6$ für $C_1$-$C_5$-Alkyl stehen.

Dabei sind diejenigen Verbindungen der Formel I bevorzugt, in denen

a) $R_1$ für $C_3$-$C_5$-Alkyl oder $C_5$-$C_6$-Cycloalkyl; $R_2$ und $R_3$ je für $C_1$-$C_4$-Alkyl; $R_4$ für Wasserstoff; $R_5$ für

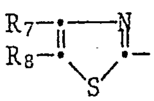 oder 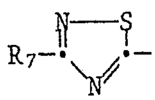 ,

wobei
$R_7$ und $R_8$ je Chlor, Brom oder Methyl bedeuten; und Z für -NH-CS-NH-stehen; oder

b) $R_1$ für $C_3$-$C_5$-Alkyl oder $C_5$-$C_6$-Cycloalkyl; $R_2$ und $R_3$ je für $C_1$-$C_4$-Alkyl; $R_4$ für Wasserstoff: $R_5$ für

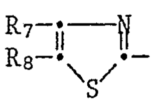 oder 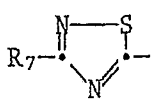 ,

wobei
$R_7$ und $R_8$ je Chlor, Brom oder Methyl bedeuten;
Z für -N = C($SR_6$)-NH-; und $R_6$ für Methyl oder Aethyl stehen; oder

c) $R_1$ für $C_3$-$C_5$-Alkyl oder $C_5$-$C_6$-Cycloalkyl; $R_2$ und $R_3$ je für $C_1$-$C_4$-Alkyl; $R_4$ für Wasserstoff; $R_5$ für

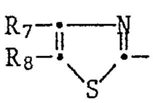 oder 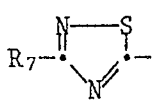 ,

wobei
$R_7$ und $R_8$ je Chlor, Brom oder Methyl bedeuten; und Z für -N = C = N-stehen.

Die erfindungsgemässen Verbindungen der Formel I können nach im Prinzip bekannten Verfahren hergestellt werden, indem man z.B.

A) ein Isothiocyanat der Formel II

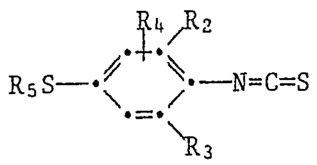

(II)

mit einem Amin der Formel III

$H_2N-R_1$ (III)

zum Thioharnstoff umsetzt und gegebenenfalls

B) den erhaltenen Thioharnstoff mit einer Verbindung der Formel IV

$X-R_6$ (IV)

zum Isothioharnstoff umsetzt oder

C) den erhaltenen Thioharnstoff durch Abspalten von Schwefelwasserstoff in das Carbodiimid überführt. $R_1$, $R_2$, $R_3$, $R_4$, $R_5$ und $R_6$ haben dabei die angegebene Bedeutung und X steht für eine geeignete Abgangsgruppe wie z.B. ein Halogenatom, insbesondere Chlor, Brom oder Jod, oder einen gegebenenfalls halogenierten oder alkylierten Sulfonsäureester, wie z.B. ein Tosylat, Brosylat oder Mono- oder Dialkylsulfat (Mesylat, Dimethylsulfat).

Das Verfahren A wird üblicherweise unter normalem Druck, und in Gegenwart eines organischen Lösungs- oder Verdünnungsmittels durchgeführt. Die Temperatur beträgt dabei zwischen 0 und +150° C, vorzugsweise +10 bis 70° C. Als Lösungs- oder Verdünnungsmittel eignen sich z.B. Aether und ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon, Methylisobutylketon oder Cyclohexanon.

Das Verfahren B wird zweckmässigerweise in einem inerten organischen Lösungsmittel und unter leicht erhöhtem oder normalem Druck durchgeführt. Die Temperatur beträgt dabei zwischen +10 und 250°C, vorzugsweise Siedetemperatur des verwendeten Lösungsmittels oder +50 bis 150° C. Geeignete Lösungs- oder Verdünnungsmittel sind z.B. Aether oder ätherartige Verbindungen, wie Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon, Alkohole oder Dimethylformamid. Die Reaktion wird entweder in Gegenwart einer Base durchgeführt oder aber das gebildete Salz anschliessend einer basischen Nachbehandlung unterworfen. (vgl. J.B. Hendricksen et al., "Organic Chemistry", Mc Graw Hill Book Co., 1970, p. 378-382).

Das Verfahren C wird zweckmässigerweise in einem aprotischen organischen Lösungs- oder Verdünnungsmittel unter normalem Druck durchgeführt. Die Temperatur beträgt dabei zwischen 0 und +150°C, vorzugsweise +10 bis 50° C. Geeignete Lösungs- oder Verdünnungsmittel sind z.B. Aether oder ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon oder Cyclohexanon. Die Abspaltung von Schwefelwasserstoff erfolgt nach in der Literatur beschriebenen Arbeitsweisen (T. Shibanuma, Chemistry Letters 1977, p. 575-76; S. Kim, Tetrahedron Letters 1985, p. 1661-64; W. Weith, B. 6, 1873, p. 1398; G. Amiard, Bull. Soc. chim. 1956, p. 1360). Als Abspaltungs-Reagenzien werden dabei u.a. HgO, bestimmte Pyridinium Salze, Chloressigsäureester, Cyanursäurechlorid, p-Toluolsulfochlorid oder bestimmte Phosphorsäureester-Derivate verwendet.

Die Isothiocyanate der Formel II können nach im Prinzip bekannten Methoden hergestellt werden, z.B. indem man ein Anilin der Formel V

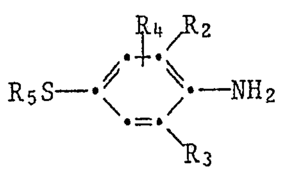

(V),

mit Thiophosgen umsetzt, wobei $R_2$, $R_3$, $R_4$ und $R_5$ die für Formel I angegebene Bedeutung haben.

Das Verfahren Zur Herstellung der Verbindungen der Formel II wird zweckmässigerweise in Gegenwart einer organischen oder anorganischen Base und einem gegenüber den Reaktionsteilnehmern inerten Lösungs-oder Verdünnungsmittels, bei einer Temperatur zwischen 0 und +100°C und bei normalem Druck durchgeführt. Geeignete Lösungs- und Verdünnungsmittel sind u.a. Aether oder ätherartige Verbindungen wie z.B. Diäthyläther, Di-isopropyläther, Dioxan oder Tetrahydrofuran; aromatische Kohlenwasserstoffe wie Benzol, Toluol oder Xylole; Ketone wie Aceton, Methyläthylketon oder Cyclohexanon; oder chlorierte Kohlenwasserstoffe, wie Dichlormethan oder Tetrachlormethan.

Geeignete Basen können organischen oder anorganischen Ursprungs sein wie z.B. Natriumhydrid, Natrium- oder Calciumcarbonat, tertiäre Amine wie Triäthylamin, Triäthylendiamin oder 4-Dimethylaminopyridin oder Pyridin.

Eine andere Möglichkeit zur Herstellung der Isothiocyanate der Formel II besteht im Umweg über den entsprechenden, einseitig unsubstituierten Thioharnstoff. Dabei wird ein Anilin der Formel V mit Ammoniumrhodanid in saurem, vorzugsweise mineralsaurem Medium zum entsprechenden Thioharnstoff umgesetzt, der seinerseits beim Erhitzen auf +130 bis 200°C Ammoniak abspaltet und in ein Isothiocyanat der Formel II übergeht (vgl. Saul Patai "The chemistry of cyanates and their thio derivatives", John Wiley and Sons, 1977, p. 1032 ff; Chemistry and Industry, July 3, 1954, p. 735. J.N. Baxter et al., "New method of preparation of aryl isothiocyanates").

Die Aniline der Formel V ihrerseits können nach im Prinzip bekannten Methoden hergestellt werden, z.B. indem man ein Mercaptoanilin der Formel VI

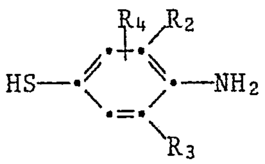

(VI)

mit einer Verbindung der Formel VII

$R_5Y$ (VII)

umsetzt, wobei $R_2$, $R_3$, $R_4$ und $R_5$ die für Formel I angegebenen Bedeutungen haben und Y für eine abspaltbare Gruppe, wie z.B. ein Halogenatom, insbesondere Chlor, Brom oder Jod steht.

Das Verfahren zur Herstellung der Thioaniline der Formel V wird zweckmässigerweise in Gegenwart einer anorganischen oder organischen Base wie z.B. einem Alkalihydroxid oder -carbonat, eines gegenüber de Reaktionsteilnehmern inerten, vorzugsweise polaren Lösungs- oder Verdünnungsmittels, und gegebenenfalls eines Katalysators wie z.B. Kupferpulver, Kupferhalogenid oder Kupfercarbonat bei einer Temperatur zwischen +80 und 160°C und unter normalem Druck durchgeführt. Geeignete Lösungs- und Verdünnungsmittel sind u.a. Aether oder ätherartige Verbindungen wie z.B. Diäthyläther, Di-isopropyläther, Dioxan oder Tetrahydrofuran; aromatische Kohlenwasserstoffe wie Benzol, Toluol oder Xylole; Ketone wie Aceton, Methyläthylketon oder Cyclohexanon; oder Alkohole wie Methanol oder Aethanol; besonders geeignet sind aprotische dipolare Lösungsmittel wie z.B. Dimethylsulfoxid oder Dimethylformamid. Wegen der Oxydationsempfindlichkeit der Aniline der Formel VI ist es vorteilhaft, die Reaktion in einer Inertgasatmosphäre durchzuführen.

Die Verbindungen der Formeln II und V sind neu und bilden ebenfalls einen Gegenstand der vorliegenden Erfindung. Die Verbindungen der Formeln II, IV, VI und VII sind dagegen bekannt oder können nach im Prinzip bekannten Methoden hergestellt werden.

Ueberraschenderweise wurde gefunden, dass die erfindungsgemässen Verbindungen der Formel I bei günstiger Warmblüter- und Pflanzenverträglichkeit wertvolle Wirkstoffe in der Schädlingsbekämpfung sind. So eignen sich die Verbindungen der Formel I z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larvan und Eier. So können vor allem Larven und Eier von phytopathogenen Schadinsekten und -milben in Zier- und Nutzpflanzungen, wie z.B. in Obst- und Gemüsepflanzungen wirkungsvoll bekämpft werden. Werden Verbindungen der Formel I von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus- und Nutztieren, kommen vor allem Ektoparasiten, wie z.B. Milben und Zecken und Dipteren, wie z.B. Lucilia sericata in Betracht.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit in der Formulierungstechnik üblichen, inerten und pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I bzw. Kombinationen dieser Wirkstoffe mit anderen Insektiziden oder Akariziden, und gegebenenfalls einen festen oder flüssigen Zusatzstoff enthal tenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"McCutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1 : Herstellung

1.1. Zwischenprodukte

1.1.1. Thioaniline

1.1.1.1. 2,6-Diisopropyl-4-(4',5'-dichlorthiazol-2'-ylthio)-anilin

26 g 2,6-Diisopropyl-4-mercapto-anilin und 22,4 g gepulvertes Kaliumcarbonat werden in 100 ml trockenem Dimethylsulfoxid vorgelegt und bei Raumtemperatur mit 23,5 g 2,4,5-Trichlorthiazol in 20 ml Dimethylsulfoxid versetzt. Die Reaktionslösung wird 24 Stunden lang bei +90°C gerührt, dann auf 500 ml Eiswasser gegossen, wiederholt mit Aether extrahiert und über Natriumsulfat getrocknet. Das Lösungsmittel wird am Vakuum entfernt und der Rückstand über Kieselgel mit einer Mischung Hexan/Essigsäureäthylester (9:1) chromatographiert. Die Titelverbindung der Formel

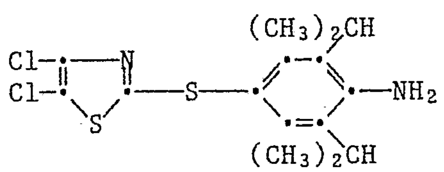

(Verb. Nr. 1.1.1.1.)

liegt als hellbrauner Feststoff vor; Smp. 100-102°C.

Auf analoge Weise werden die folgenden Verbindungen hergestellt:

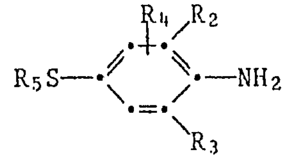

| Verb.Nr. | $R_2$ | $R_3$ | $R_4$ | $R_5$* | $R_7$ | $R_8$ | $R_9$ | phys. Daten |
|---|---|---|---|---|---|---|---|---|
| 1.1.1.2. | $CH(CH_3)_2$ | $CH(CH_3)_2$ | H | $Q_1'$ | Cl | Br | – | Smp.120-123°C |
| 1.1.1.3. | $CH(CH_3)_2$ | $CH(CH_3)_2$ | H | $Q_4$ | $CH_3$ | – | – | Smp.112-114°C |
| 1.1.1.4. | $C_2H_5$ | $C_2H_5$ | H | $Q_4$ | $CH_3$ | – | – | Smp.139-141°C |
| 1.1.1.5. | $CH(CH_3)_2$ | $CH(CH_3)_2$ | H | $Q_1'$ | Cl | $CH_3$ | – | Smp. 94-96°C |
| 1.1.1.6. | $C_2H_5$ | $C_2H_5$ | H | $Q_1'$ | Cl | Cl | – | m/e = 332/334 |
| 1.1.1.7. | $C_2H_5$ | $CH(CH_3)C_2H_5$ | H | $Q_4$ | $CH_3$ | – | – | Smp. 53- 55°C |
| 1.1.1.8. | $CH(CH_3)_2$ | $CH(CH_3)_2$ | H | $Q_4$ | $CH(CH_3)_2$ | – | – | m/e = 335 |
| 1.1.1.9. | $CH(CH_3)_2$ | $CH(CH_3)_2$ | H | $Q_7$ | 6-Cl | – | – | Smp.117-119°C |
| 1.1.1.10. | $C_2H_5$ | $CH(CH_3)_2$ | H | $Q_1'$ | Cl | Cl | – | m/e = 346/348 |
| 1.1.1.11. | $CH(CH_3)_2$ | $CH(CH_3)_2$ | H | $Q_8$ | H | H | – | Smp.133-135°C |
| 1.1.1.12. | $CH(CH_3)_2$ | $CH(CH_3)_2$ | H | $Q_8$ | 6-Cl | H | – | Smp.108-110°C |
| 1.1.1.13. | $CH(CH_3)_2$ | $CH(CH_3)_2$ | H | $Q_8$ | 4-Cl | H | – | Smp.179,5-181,5°C |
| | $CH_3$ | $CH_3$ | H | $Q_2$ | $CF_3$ | – | $CH(CH_3)_2$ | |
| | $CH(CH_3)_2$ | $CH(CH_3)_2$ | H | $Q_2$ | $CF_3$ | – | $CH(CH_3)_2$ | |
| | $CH(CH_3)_2$ | $CH(CH_3)_2$ | H | $Q_3$ | $CF_3$ | – | – | |
| | Cyclopentyl | $CH(CH_3)_2$ | H | $Q_1'$ | Cl | Cl | – | |

* $Q_1'$

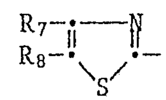

$Q_2$

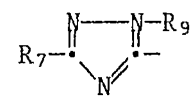

$Q_3$

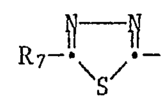

$Q_4$

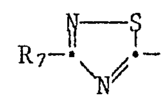

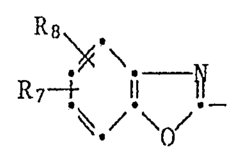

$Q_7$

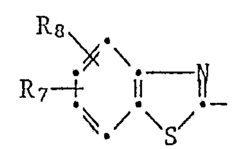

$Q_8$

1.1.2. Isothiocyanate

1.1.2.1. 2,6 Diisopropyl-4-(4′,5′-dichlorthiazol-2′-ylthio)-phenyl isothiocyanat

4,2 g Thiophosgen, 6 g Calciumcarbonat und 70 ml Dichlormethan werden mit 45 ml Wasser verrührt. Zu diesem Gemisch wird bei Raumtemperatur tropfenweise unter Rühren eine Lösung von 10,8 g 2,6-Diisopropyl-4-(4′,5′-dichlorthiazol-2′-ylthio)-anilin in 20 ml Dichlormethan gegeben. Das Reaktionsgemisch wird 2 Stunden lang unter Rückfluss gerührt und nach dem Erkalten filtriert. Aus dem Filtrat wird die organische Phase abgetrennt und die wässrige Phase mit Dichlormethan gewaschen; die vereinigten organischen Phasen werden mit Wasser gewaschen, über Natriumsulfat getrocknet, und schliesslich wird das Lösungsmittel abdestilliert. Die Titelverbindung der Formel

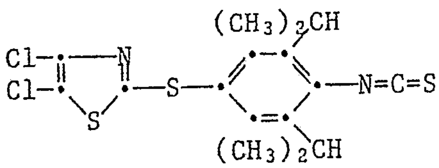

(Verb. Nr. 1.1.2.1.)

liegt in Form eines gelben Oels vor, das ohne weitere Reinigung für die nächste Reaktion verwendet wird.

Auf analoge Weise werden die folgenden Verbindungen hergestellt:

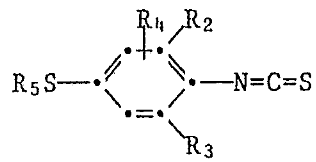

| Verb.Nr. | $R_2$ | $R_3$ | $R_4$ | $R_5$* | $R_7$ | $R_8$ | $R_9$ | phys. Daten |
|---|---|---|---|---|---|---|---|---|
| 1.1.2.2. | $CH(CH_3)_2$ | $CH(CH_3)_2$ | H | $Q_1'$ | Cl | Br | - | brauner Feststoff |
| 1.1.2.3. | $CH(CH_3)_2$ | $CH(CH_3)_2$ | H | $Q_4$ | $CH_3$ | - | - | Smp. 92-93°C |
| 1.1.2.4. | $C_2H_5$ | $C_2H_5$ | H | $Q_4$ | $CH_3$ | - | - | Smp. 35-37°C |
| 1.1.2.5. | $CH(CH_3)_2$ | $CH(CH_3)_2$ | H | $Q_1'$ | Cl | $CH_3$ | - | Smp. 75-78°C |
| 1.1.2.6. | $C_2H_5$ | $C_2H_5$ | H | $Q_1'$ | Cl | Cl | - | gelber Feststoff |
| 1.1.2.7. | $C_2H_5$ | $CH(CH_3)C_2H_5$ | H | $Q_4$ | $CH_3$ | - | - | $n_D^{25}$: 1,658 |
| 1.1.2.8. | $CH(CH_3)_2$ | $CH(CH_3)_2$ | H | $Q_4$ | $CH(CH_3)_2$ | - | - | Smp. 30°C |
| 1.1.2.9. | $CH(CH_3)_2$ | $CH(CH_3)_2$ | H | $Q_7$ | 6-Cl | - | - | Smp. 82- 84°C |
| 1.1.2.10. | $C_2H_5$ | $CH(CH_3)_2$ | H | $Q_1'$ | Cl | Cl | - | gelber Feststoff |
| 1.1.2.11. | $CH(CH_3)_2$ | $CH(CH_3)_2$ | H | $Q_8$ | H | H | - | Smp.122-124,5°C |
| 1.1.2.12. | $CH(CH_3)_2$ | $CH(CH_3)_2$ | H | $Q_8$ | 6-Cl | H | - | Smp.168-169°C |
| 1.1.2.13. | $CH(CH_3)_2$ | $CH(CH_3)_2$ | H | $Q_8$ | 4-Cl | H | - | hellgelber Feststoff |
| | $CH_3$ | $CH_3$ | H | $Q_2$ | $CF_3$ | - | $CH(CH_3)_2$ | |
| | $CH(CH_3)_2$ | $CH(CH_3)_2$ | H | $Q_2$ | $CF_3$ | - | $CH(CH_3)_2$ | |
| | $CH(CH_3)_2$ | $CH(CH_3)_2$ | H | $Q_3$ | $CF_3$ | - | - | |
| | Cyclopentyl | $CH(CH_3)_2$ | H | $Q_1'$ | Cl | Cl | - | |

* $Q_1'$

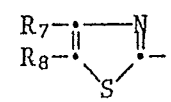

$Q_2$

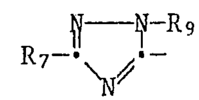

$Q_3$

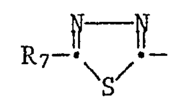

$Q_4$

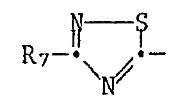

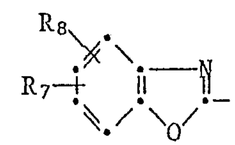

$Q_7$

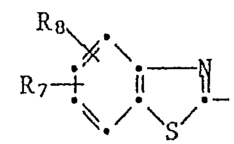

$Q_8$

## 1.2. Endprodukte

### 1.2.1. Phenylthioharnstoffe

#### 1.2.1.1. N-[2,6-Diisopropyl-4-(4',5'-dichlorthiazol-2'-ylthio)-phenyl]-N'-tert.butyl-thioharnstoff

7,7 g 2,6-Diisopropyl-4-(4',5'-dichlorthiazol-2'-ylthio)-phenylisothiocyanat und 3,0 g tert.Butylamin werden mit 50 ml Tetrahydrofuran verdünnt und 6 Stunden lang auf +40°C erwärmt. Das Reaktionsgemisch wird auf 300 ml Eiswasser gegossen; der entstandene Niederschlag abfiltriert und aus Hexan/Toluol umkristallisiert. Die Titelverbindung der Formel

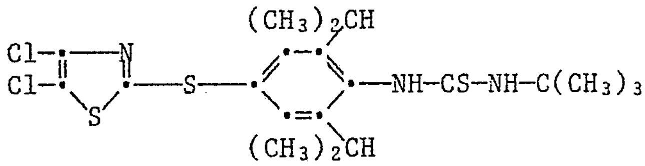

(Verb. Nr. 1.2.1.1.)

liegt in Form farbloser Kristalle vor; Smp. 153-154°C.

Auf analoge Weise werden die folgenden Verbindungen hergestellt:

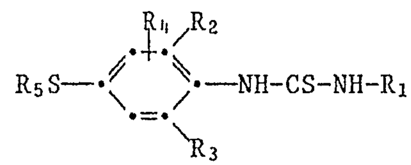

| Verb.Nr. | $R_1$ | $R_2$ | $R_3$ | $R_4$ | $R_5$* | $R_7$ | $R_8$ | $R_9$ | Smp. °C |
|---|---|---|---|---|---|---|---|---|---|
| 1.2.1.2. | $C(CH_3)_3$ | $CH(CH_3)_2$ | $CH(CH_3)_2$ | H | $Q_1'$ | Cl | Br | - | 172-173 |
| 1.2.1.3. | $C(CH_3)_3$ | $CH(CH_3)_2$ | $CH(CH_3)_2$ | H | $Q_4$ | $CH_3$ | - | - | 146-148 |
| 1.2.1.4. | $C(CH_3)_3$ | $C_2H_5$ | $C_2H_5$ | H | $Q_4$ | $CH_3$ | - | - | 108-110 |
| 1.2.1.5. | $CH(CH_3)_2$ | $C_2H_5$ | $C_2H_5$ | H | $Q_4$ | $CH_3$ | - | - | 142-144 |
| 1.2.1.6. | $CH(CH_3)C_2H_5$ | $C_2H_5$ | $C_2H_5$ | H | $Q_4$ | $CH_3$ | - | - | 97- 99 |
| 1.2.1.7. | $C(CH_3)_3$ | $CH(CH_3)_2$ | $CH(CH_3)_2$ | H | $Q_1'$ | Cl | $CH_3$ | - | 160-162 |
| 1.2.1.8. | $CH(CH_3)_2$ | $CH(CH_3)_2$ | $CH(CH_3)_2$ | H | $Q_1'$ | Cl | $CH_3$ | - | 158-161 |
| 1.2.1.9. | Cyclopentyl | $CH(CH_3)_2$ | $CH(CH_3)_2$ | H | $Q_1'$ | Cl | $CH_3$ | - | 163,5-166 |
| 1.2.1.10. | $C(CH_3)_3$ | $C_2H_5$ | $C_2H_5$ | H | $Q_1'$ | Cl | Cl | - | 135,5-139 |
| 1.2.1.11. | Cyclopentyl | $C_2H_5$ | $C_2H_5$ | H | $Q_4$ | $CH_3$ | - | - | 148-150 |
| 1.2.1.12. | $CH(CH_3)_2$ | $CH(CH_3)_2$ | $CH(CH_3)_2$ | H | $Q_4$ | $CH_3$ | - | - | 118-119 |
| 1.2.1.13. | $CH(CH_3)C_2H_5$ | $CH(CH_3)_2$ | $CH(CH_3)_2$ | H | $Q_4$ | $CH_3$ | - | - | 118-119 |
| 1.2.1.14. | $C(CH_3)_2C_2H_5$ | $CH(CH_3)_2$ | $CH(CH_3)_2$ | H | $Q_4$ | $CH_3$ | - | - | 142-144 |
| 1.2.1.15. | Cyclopentyl | $CH(CH_3)_2$ | $CH(CH_3)_2$ | H | $Q_4$ | $CH_3$ | - | - | 154-156 |
| 1.2.1.16. | $C(CH_3)_3$ | $C_2H_5$ | $CH(CH_3)C_2H_5$ | H | $Q_4$ | $CH_3$ | - | - | 112-114 |
| 1.2.1.17. | $CH(CH_3)_2$ | $C_2H_5$ | $CH(CH_3)C_2H_5$ | H | $Q_4$ | $CH_3$ | - | - | 78- 80 |
| 1.2.1.18. | $C(CH_3)_3$ | $CH(CH_3)_2$ | $CH(CH_3)_2$ | H | $Q_4$ | $CH(CH_3)_2$ | - | - | 144-146 |
| 1.2.1.19. | $CH(CH_3)_2$ | $C_2H_5$ | $C_2H_5$ | H | $Q_1'$ | Cl | Cl | - | 146-147 |
| 1.2.1.20. | $C(CH_3)_3$ | $CH(CH_3)_2$ | $C_2H_5$ | H | $Q_1'$ | Cl | Cl | - | 132-138 |
| 1.2.1.21. | $CH(CH_3)_2$ | $CH(CH_3)_2$ | $C_2H_5$ | H | $Q_1'$ | Cl | Cl | - | 159-162 |
| 1.2.1.22. | $C(CH_3)_3$ | $CH(CH_3)_2$ | $CH(CH_3)_2$ | H | $Q_7$ | 6-Cl | H | - | 134 |
| 1.2.1.23. | $C(CH_3)_3$ | $CH(CH_3)_2$ | $CH(CH_3)_2$ | H | $Q_8$ | H | H | - | 142-144 |

| Verb.Nr. | $R_1$ | $R_2$ | $R_3$ | $R_4$ | $R_5$* | $R_7$ | $R_8$ | $R_9$ | Smp. °C |
|---|---|---|---|---|---|---|---|---|---|
| 1.2.1.24. | $CH(CH_3)_2$ | $CH(CH_3)_2$ | $CH(CH_3)_2$ | H | $Q_8$ | H | H | - | 178-181 |
| 1.2.1.25. | $C(CH_3)_3$ | $CH(CH_3)_2$ | $CH(CH_3)_2$ | H | $Q_8$ | 4-Cl | H | - | 153,5-155,5 |
| 1.2.1.26. | $CH(CH_3)_2$ | $CH(CH_3)_2$ | $CH(CH_3)_2$ | H | $Q_8$ | 6-Cl | H | - | 181-183,5 |
| 1.2.1.27. | $C(CH_3)_3$ | $CH(CH_3)_2$ | $CH(CH_3)_2$ | H | $Q_8$ | 6-Cl | H | - | 152-153 |
| | $C(CH_3)_3$ | $CH_3$ | $CH_3$ | H | $Q_2$ | $CF_3$ | - | $CH(CH_3)_2$ | |
| | $C(CH_3)_3$ | $CH(CH_3)_2$ | $CH(CH_3)_2$ | H | $Q_2$ | $CF_3$ | - | $CH(CH_3)_2$ | |
| | $C(CH_3)_3$ | $CH(CH_3)_2$ | $CH(CH_3)_2$ | H | $Q_3$ | $CF_3$ | - | - | |
| | Cyclopentyl | $CH(CH_3)_2$ | $CH(CH_3)_2$ | H | $Q_1'$ | Cl | Cl | - | |
| | Cyclopentyl | $CH(CH_3)_2$ | $CH(CH_3)_2$ | H | $Q_1'$ | $CH_3$ | - | - | |
| | $C(CH_3)_3$ | Cyclopentyl | $CH(CH_3)_2$ | H | $Q_1'$ | Cl | Cl | - | |

* $Q_1'$ 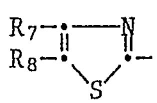 $Q_2$ 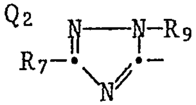 $Q_3$ 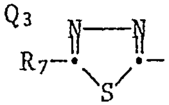 $Q_4$ 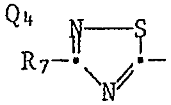 $Q_7$ 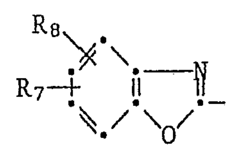 $Q_8$ 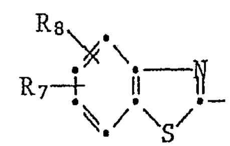

1.2.2. Phenylisothioharnstoffe

1.2.2.1. N-[2,6-Diisopropyl-4-(4',5'-dichlorthiazol-2'-ylthio)-phenyl]-N'-(tert.butyl)-S-methyl-isothioharnstoff

3,6 g N-[2,6-Diisopropyl-4-(4',5'-dichlorthiazol-2'-ylthio)-phenyl]-N'-tert.butyl-thioharnstoff in 40 ml Aethanol werden bei Raumtemperatur mit 1,6 g Methyljodid versetzt und 6 Stunden lang auf +50°C erwärmt. Anschliessend wird die Reaktionslösung eingedampft, der Rückstand in Methylenchlorid aufgenommen und zweimal mit verdünnter Natriumcarbonatlösung gewaschen. Die organische Phase wird über Natriumsulfat getrocknet und das Lösungsmittel abgedampft. Die Titelverbindung der Formel

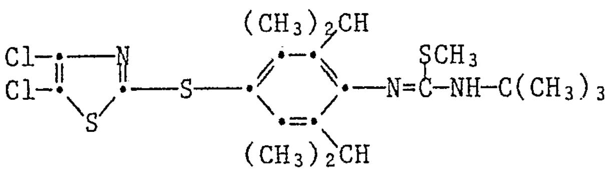

(Verb. Nr. 1.2.2.1.)

liegt in Form eines farblosen Kristallpulvers vor; Smp. 116-117°C.

Auf analoge Weise werden die folgenden Verbindungen hergestellt:

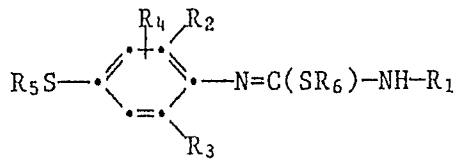

| Verb.Nr. | $R_1$ | $R_2$ | $R_3$ | $R_4$ | $R_5$* | $R_6$ | $R_7$ | $R_8$ | $R_9$ | phys. Daten |
|---|---|---|---|---|---|---|---|---|---|---|
| 1.2.2.2. | $C(CH_3)_3$ | $CH(CH_3)_2$ | $CH(CH_3)_2$ | H | $Q_1'$ | $CH_3$ | Cl | Br | – | Smp. 118,5-119,5°C |
| 1.2.2.3. | $C(CH_3)_3$ | $CH(CH_3)_2$ | $CH(CH_3)_2$ | H | $Q_4$ | $CH_3$ | $CH_3$ | – | – | Smp. 114-116°C |
| 1.2.2.4. | $C(CH_3)_3$ | $C_2H_5$ | $C_2H_5$ | H | $Q_4$ | $C_2H_5$ | $CH_3$ | – | – | Smp. 82- 84°C |
| 1.2.2.5. | $CH(CH_3)_2$ | $C_2H_5$ | $C_2H_5$ | H | $Q_4$ | $CH_3$ | $CH_3$ | – | – | $n_D^{40}$: 1,5984 |
| 1.2.2.6. | $CH(CH_3)C_2H_5$ | $C_2H_5$ | $C_2H_5$ | H | $Q_4$ | $CH_3$ | $CH_3$ | – | – | $n_D^{25}$: 1,5934 |
| 1.2.2.7. | $C(CH_3)_3$ | $CH(CH_3)_2$ | $CH(CH_3)_2$ | H | $Q_1'$ | $CH_3$ | Cl | $CH_3$ | – | Smp. 95- 98°C |
| 1.2.2.8. | $CH(CH_3)_2$ | $CH(CH_3)_2$ | $CH(CH_3)_2$ | H | $Q_1'$ | $CH_3$ | Cl | $CH_3$ | – | Smp. 93- 95°C |
| 1.2.2.9. | Cyclopentyl | $CH(CH_3)_2$ | $CH(CH_3)_2$ | H | $Q_1'$ | $CH_3$ | Cl | $CH_3$ | – | Smp. 101,5-102,5°C |
| 1.2.2.10. | $C(CH_3)_3$ | $C_2H_5$ | $C_2H_5$ | H | $Q_1'$ | $CH_3$ | Cl | Cl | – | Smp. 61,5-63,5°C |
| 1.2.2.11. | Cyclopentyl | $C_2H_5$ | $C_2H_5$ | H | $Q_4$ | $CH_3$ | $CH_3$ | – | – | $n_D^{40}$: 1,5995 |
| 1.2.2.12. | $CH(CH_3)C_2H_5$ | $CH(CH_3)_2$ | $CH(CH_3)_2$ | H | $Q_4$ | $CH_3$ | $CH_3$ | – | – | $n_D^{40}$: 1,5796 |
| 1.2.2.13. | $C(CH_3)_2C_2H_5$ | $CH(CH_3)_2$ | $CH(CH_3)_2$ | H | $Q_4$ | $CH_3$ | $CH_3$ | – | – | Smp. 96-98°C |
| 1.2.2.14. | $CH(CH_3)_2$ | $C_2H_5$ | $CH(CH_3)C_2H_5$ | H | $Q_4$ | $CH_3$ | $CH_3$ | – | – | $n_D^{40}$: 1,5875 |
| 1.2.2.15. | Cyclopentyl | $CH(CH_3)_2$ | $CH(CH_3)_2$ | H | $Q_4$ | $CH_3$ | $CH_3$ | – | – | Smp.114-116°C |
| 1.2.2.16. | $C(CH_3)_3$ | $CH(CH_3)_2$ | $CH(CH_3)_2$ | H | $Q_4$ | $CH_3$ | $CH(CH_3)_2$ | – | – | Smp. 91- 93°C |
| 1.2.2.17. | $CH(CH_3)_2$ | $C_2H_5$ | $C_2H_5$ | H | $Q_1'$ | $CH_3$ | Cl | Cl | – | Smp. 78- 80°C |
| 1.2.2.18. | $C(CH_3)_3$ | $CH(CH_3)_2$ | $C_2H_5$ | H | $Q_1'$ | $CH_3$ | Cl | Cl | – | $n_D^{39}$: 1,6037 |
| 1.2.2.19. | $CH(CH_3)_2$ | $CH(CH_3)_2$ | $C_2H_5$ | H | $Q_1'$ | $CH_3$ | Cl | Cl | – | $n_D^{39}$: 1,5991 |
| 1.2.2.20. | $C(CH_3)_3$ | $CH(CH_3)_2$ | $CH(CH_3)_2$ | H | $Q_8$ | $CH_3$ | H | H | – | Smp.166,5-168°C |
| 1.2.2.21. | $CH(CH_3)_2$ | $CH(CH_3)_2$ | $CH(CH_3)_2$ | H | $Q_8$ | $CH_3$ | H | H | – | Smp.108-111°C |

| Verb.Nr. | $R_1$ | $R_2$ | $R_3$ | $R_4$ | $R_5$* | $R_6$ | $R_7$ | $R_8$ | $R_9$ | phys. Daten |
|---|---|---|---|---|---|---|---|---|---|---|
| 1.2.2.22. | $C(CH_3)_3$ | $CH(CH_3)_2$ | $CH(CH_3)_2$ | H | $Q_8$ | $CH_3$ | 4-Cl | H | - | Smp.175-176°C |
| 1.2.2.23. | $CH(CH_3)_2$ | $CH(CH_3)_2$ | $CH(CH_3)_2$ | H | $Q_8$ | $CH_3$ | 6-Cl | H | - | Smp.105,5-107,5°C |
| 1.2.2.24. | $C(CH_3)_3$ | $CH(CH_3)_2$ | $CH(CH_3)_2$ | H | $Q_8$ | $CH_3$ | 6-Cl | H | - | Smp.168-172°C |
| 1.2.2.25. | $CH(CH_3)_2$ | $CH(CH_3)_2$ | $CH(CH_3)_2$ | H | $Q_4$ | $CH_3$ | $CH_3$ | - | - | $n_D^{40}$: 1,5847 |
| | $C(CH_3)_3$ | $CH_3$ | $CH_3$ | H | $Q_2$ | $CH_3$ | $CF_3$ | - | $CH(CH_3)_2$ | |
| | $C(CH_3)_3$ | $CH(CH_3)_2$ | $CH(CH_3)_2$ | H | $Q_2$ | $CH_3$ | $CF_3$ | - | $CH(CH_3)_2$ | |
| | $CH(CH_3)_2$ | $CH(CH_3)_2$ | $CH(CH_3)_2$ | H | $Q_4$ | $CH_3$ | $CH_3$ | - | - | |
| | $C(CH_3)_3$ | $CH(CH_3)_2$ | $CH(CH_3)_2$ | H | $Q_3$ | $CH_3$ | $CF_3$ | - | - | |
| | Cyclopentyl | $CH(CH_3)_2$ | $CH(CH_3)_2$ | H | $Q_1'$ | $CH_3$ | Cl | Cl | - | |
| | Cyclopentyl | $CH(CH_3)_2$ | $CH(CH_3)_2$ | H | $Q_1'$ | $CH_3$ | $CH_3$ | - | - | |
| | $C(CH_3)_3$ | Cyclopentyl | $CH(CH_3)_2$ | H | $Q_1'$ | $CH_3$ | Cl | Cl | - | |
| | $C(CH_3)_3$ | $C_2H_5$ | $CH(CH_3)C_2H_5$ | H | $Q_4$ | $CH_3$ | $CH_3$ | - | - | |

* $Q_1'$ 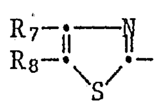 $Q_2$ 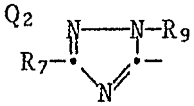 $Q_3$ 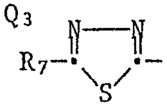 $Q_4$ 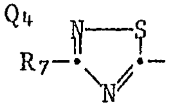 $Q_7$ 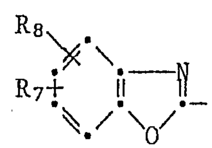 $Q_8$ 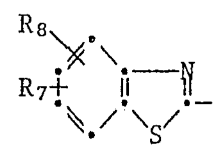

1.2.3. Phenylisothioharnstoff-Salze

1.2.3.1. N-[2,6-Diisopropyl-4-(4′-chlor-5′-methylthiazol-2′-ylthio)-phenyl)-N′-isopropyl-S-methyl-isothioharnstoff Jodwasserstoffsäuresalz

5 g N-[2,6-Diisopropyl-4-(4′-chlor-5′-methylthiazol-2′-ylthio)-phenyl]-N′-isopropyl-S-methyl-isothioharnstoff werden in 20 ml absolutem Diäthyläther gelöst und tropfenweise mit 1 g Jodwasserstoffsäure versetzt. Die Reaktionslösung wird 3 Stunden lang bei Raumtemperatur nachgerührt. Der entstandene Niederschlag wird abgenutscht und getrocknet. Die Titelverbindung der Formel

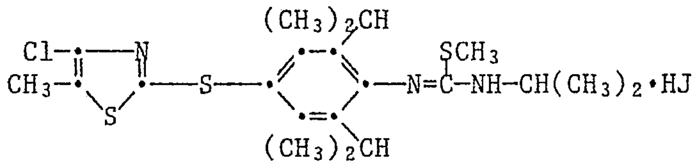 (Verb. Nr. 1.2.3.1.)

liegt als farbloses Kristallpulver vor; Smp. 180-183° C.

Auf analoge Weise werden die folgenden Verbindungen hergestellt:

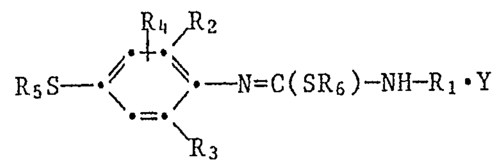

| Verb.Nr. | $R_1$ | $R_2$ | $R_3$ | $R_4$ | $R_5$* | $R_6$ | $R_7$ | $R_8$ | $R_9$ | Y | Smp. °C |
|---|---|---|---|---|---|---|---|---|---|---|---|
| 1.2.3.2. | Cyclopentyl | $CH(CH_3)_2$ | $CH(CH_3)_2$ | H | $Q_1'$ | $CH_3$ | Cl | $CH_3$ | – | HJ | 167-170 |
| 1.2.3.3. | $CH(CH_3)_2$ | $CH(CH_3)_2$ | $C_2H_5$ | H | $Q_1'$ | $CH_3$ | Cl | Cl | – | HJ | 152-159 |
| 1.2.3.4. | $C(CH_3)_3$ | $CH(CH_3)_2$ | $CH(CH_3)_2$ | H | $Q_8$ | $CH_3$ | H | H | – | HJ | 157-160 |
| 1.2.3.5. | $CH(CH_3)_2$ | $CH(CH_3)_2$ | $CH(CH_3)_2$ | H | $Q_8$ | $CH_3$ | H | H | – | HJ | 159-163 |
| 1.2.3.6. | $CH(CH_3)_2$ | $CH(CH_3)_2$ | $CH(CH_3)_2$ | H | $Q_8$ | $CH_3$ | 6-Cl | H | – | HJ | 174-180 |

* $Q_1'$ 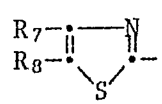

$Q_8$ 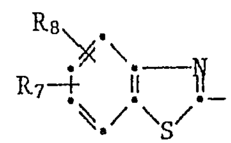

1.2.4. Phenylcarbodiimide

1.2.4.1. N-[2,6-Diisopropyl-4-(4′,5′-dichlorthiazol-2′-ylthio)-phenyl]-N′-tert.butyl-carbodiimid

4,2 g N-[2,6-Diisopropyl-4-(4′,5′-dichlorthiazol-2′-ylthio)-phenyl]-N′-tert.butyl-thioharnstoff und 2,9 g 2-Chlor-1-methylpyridiniumjodid werden in 40 ml trockenem Acetonitril vorgelegt, bei Raumtemperatur tropfenweise mit 2,3 g Triäthylamin in 50 ml Acetonitril versetzt und während 3 Stunden unter Rückfluss gerührt. Anschliessend wird das Lösungsmittel am Vakuum entfernt, der Rückstand in Hexan aufgenommen und filtriert. Das Filtrat wird dreimal mit Wasser gewaschen, über Natriumsulfat getrocknet und am Vakuum vom Lösungsmittel befreit. Die Titelverbindung der Formel

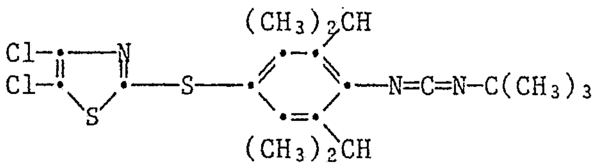

(Verb. Nr. 1.2.4.1.)

liegt als farbloses Kristallpulver vor; Smp. 60,5-61,5°C.

Auf analoge Weise werden die folgenden Verbindungen hergestellt:

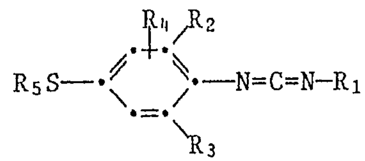

| Verb.Nr. | $R_1$ | $R_2$ | $R_3$ | $R_4$ | $R_5$* | $R_7$ | $R_8$ | $R_9$ | phys. Daten |
|---|---|---|---|---|---|---|---|---|---|
| 1.2.4.2. | $C(CH_3)_3$ | $CH(CH_3)_2$ | $CH(CH_3)_2$ | H | $Q_1'$ | Cl | Br | – | Smp. 60,5-61,5°C |
| 1.2.4.3. | $C(CH_3)_3$ | $CH(CH_3)_2$ | $CH(CH_3)_2$ | H | $Q_4$ | $CH_3$ | – | – | $n_D^{40}$: 1,5730 |
| 1.2.4.4. | $C(CH_3)_3$ | $C_2H_5$ | $C_2H_5$ | H | $Q_4$ | $CH_3$ | – | – | $n_D^{40}$: 1,5897 |
| 1.2.4.5. | $CH(CH_3)_2$ | $C_2H_5$ | $C_2H_5$ | H | $Q_4$ | $CH_3$ | – | – | $n_D^{40}$: 1,5926 |
| 1.2.4.6. | $C(CH_3)_3$ | $CH(CH_3)_2$ | $CH(CH_3)_2$ | H | $Q_1'$ | Cl | $CH_3$ | – | Smp. 82-83,5°C |
| 1.2.4.7. | $CH(CH_3)_2$ | $CH(CH_3)_2$ | $CH(CH_3)_2$ | H | $Q_1'$ | Cl | $CH_3$ | – | Smp. 69-71°C |
| 1.2.4.8. | Cyclopentyl | $CH(CH_3)_2$ | $CH(CH_3)_2$ | H | $Q_1'$ | Cl | $CH_3$ | – | Smp. 69-71°C |
| 1.2.4.9. | $C(CH_3)_3$ | $C_2H_5$ | $C_2H_5$ | H | $Q_1'$ | Cl | Cl | – | Smp. 62-65°C |
| 1.2.4.10. | $CH(CH_3)C_2H_5$ | $C_2H_5$ | $C_2H_5$ | H | $Q_4$ | $CH_3$ | – | – | $n_D^{25}$: 1,5934 |
| 1.2.4.11. | Cyclopentyl | $C_2H_5$ | $C_2H_5$ | H | $Q_4$ | $CH_3$ | – | – | $n_D^{25}$: 1,6095 |
| 1.2.4.12. | $CH(CH_3)_2$ | $CH(CH_3)_2$ | $CH(CH_3)_2$ | H | $Q_4$ | $CH_3$ | – | – | $n_D^{25}$: 1,5860 |
| 1.2.4.13. | $CH(CH_3)C_2H_5$ | $CH(CH_3)_2$ | $CH(CH_3)_2$ | H | $Q_4$ | $CH_3$ | – | – | $n_D^{25}$: 1,5820 |
| 1.2.4.14. | $C(CH_3)_2C_2H_5$ | $CH(CH_3)_2$ | $CH(CH_3)_2$ | H | $Q_4$ | $CH_3$ | – | – | $n_D^{25}$: 1,5765 |
| 1.2.4.15. | $C(CH_3)_3$ | $C_2H_5$ | $CH(CH_3)C_2H_5$ | H | $Q_4$ | $CH_3$ | – | – | $n_D^{25}$: 1,5793 |
| 1.2.4.16. | $CH(CH_3)_2$ | $C_2H_5$ | $CH(CH_3)C_2H_5$ | H | $Q_4$ | $CH_3$ | – | – | $n_D^{25}$: 1,5865 |
| 1.2.4.17. | Cyclopentyl | $CH(CH_3)_2$ | $CH(CH_3)_2$ | H | $Q_4$ | $CH_3$ | – | – | $n_D^{25}$: 1,5955 |
| 1.2.4.18. | $C(CH_3)_3$ | $CH(CH_3)_2$ | $CH(CH_3)_2$ | H | $Q_4$ | $CH(CH_3)_2$ | – | – | Smp. 50-52°C |

| Verb.Nr. | $R_1$ | $R_2$ | $R_3$ | $R_4$ | $R_5$* | $R_7$ | $R_8$ | $R_9$ | phys. Daten |
|---|---|---|---|---|---|---|---|---|---|
| 1.2.4.19. | $CH(CH_3)_2$ | $C_2H_5$ | $C_2H_5$ | H | $Q_1'$ | Cl | Cl | - | $n_D^{24}$ : 1,6183 |
| 1.2.4.20. | $C(CH_3)_3$ | $CH(CH_3)_2$ | $C_2H_5$ | H | $Q_1'$ | Cl | Cl | - | $n_D^{24}$ : 1,6040 |
| 1.2.4.21. | $CH(CH_3)_2$ | $CH(CH_3)_2$ | $C_2H_5$ | H | $Q_1'$ | Cl | Cl | - | $n_D^{24}$ : 1,6121 |
| 1.2.4.22. | $C(CH_3)_3$ | $CH(CH_3)_2$ | $CH(CH_3)_2$ | H | $Q_7$ | 6-Cl | H | - | $n_D^{25}$ : 1,5992 |
| 1.2.4.23. | $C(CH_3)_3$ | $CH(CH_3)_2$ | $CH(CH_3)_2$ | H | $Q_8$ | H | H | - | Smp.80,5-82°C |
| 1.2.4.24. | $CH(CH_3)_2$ | $CH(CH_3)_2$ | $CH(CH_3)_2$ | H | $Q_8$ | H | H | - | Smp.62-65,5°C |
| 1.2.4.25. | $C(CH_3)_3$ | $CH(CH_3)_2$ | $CH(CH_3)_2$ | H | $Q_8$ | 4-Cl | H | - | Smp.133-134°C |
| 1.2.4.26. | $CH(CH_3)_2$ | $CH(CH_3)_2$ | $CH(CH_3)_2$ | H | $Q_8$ | 6-Cl | H | - | Smp.83-86°C |
| 1.2.4.27. | $C(CH_3)_3$ | $CH(CH_3)_2$ | $CH(CH_3)_2$ | H | $Q_8$ | 6-Cl | H | - | Smp.104-107,5°C |
| | $C(CH_3)_3$ | $CH_3$ | $CH_3$ | H | $Q_2$ | $CF_3$ | - | $CH(CH_3)_2$ | |
| | $C(CH_3)_3$ | $CH(CH_3)_2$ | $CH(CH_3)_2$ | H | $Q_2$ | $CF_3$ | - | $CH(CH_3)_2$ | |
| | $C(CH_3)_3$ | $CH(CH_3)_2$ | $CH(CH_3)_2$ | H | $Q_3$ | $CF_3$ | - | - | |
| | Cyclopentyl | $CH(CH_3)_2$ | $CH(CH_3)_2$ | H | $Q_1'$ | Cl | Cl | - | |
| | Cyclopentyl | $CH(CH_3)_2$ | $CH(CH_3)_2$ | H | $Q_1'$ | $CH_3$ | - | - | |
| | $C(CH_3)_3$ | Cyclopentyl | $CH(CH_3)_2$ | H | $Q_1'$ | Cl | Cl | - | |

* $Q_1'$ 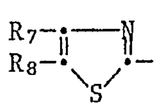 $Q_2$ 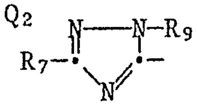 $Q_3$ 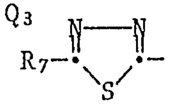 $Q_4$ 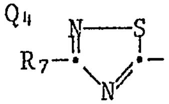 $Q_7$ 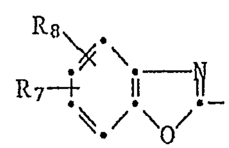 $Q_8$ 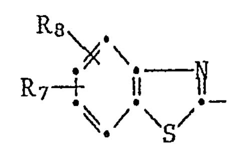

Beispiel 2: Formulierungen von Wirkstoffen der Formel I gemäss den Herstellungsbeispielen 1.2.

(% = Gewichtsprozent)

| 2.1. Emulsions-Konzentrate | a) | b) |
|---|---|---|
| Wirkstoff gemäss den Herstellungsbeispielen 1.2. | 10 % | 25 % |
| Ca-Dodecylbenzolsulfonat | - | 5 % |
| Ricinusöl-polyäthylenglykoläther (36 Mol AeO) | 25 % | 5 % |
| Cyclohexanon | - | 40 % |
| Butanol | 15 % | - |
| Xylolgemisch | - | 25 % |
| Essigester | 50 % | - |

Aus solchen Konzentraten können durch Verdünnen mit Wasser Emulsionen jeder gewünschten Konzentration hergestellt werden.

| 2.2. Lösungen | a) | b) |
|---|---|---|
| Wirkstoff gemäss den Herstellungsbeispielen 1.2. | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190°C) | - | 74 % |

Die Lösungen sind zur Anwendung in Form kleinster Tropfen geeignet.

| 2.3. Granulate | a) | b) |
|---|---|---|
| Wirkstoff gemäss den Herstellungsbeispielen 1.2. | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4 Extruder Granulat
Wirkstoff gemäss den Herstellungsbeispielen 1.2. 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

2.5. Umhüllungs-Granulat
Wirkstoff gemäss den Herstellungsbeispielen 1.2. 3 %
Polyäthylenglykol (MG 200) 3 %
Kaolin 94 %

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 2.6. Stäubemittel | a) | b) | c) | d) |
|---|---|---|---|---|
| Wirkstoff gemäss den Herstellungsbeispielen 1.2. | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| 2.7. Spritzpulver | a) | b) | c) |
|---|---|---|---|
| Wirkstoff gemäss den Herstellungsbeispielen 1.2. | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

2.8. Suspensions-Konzentrat
Wirkstoff gemäss den Herstellungsbeispielen 1.2. 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0.8 %
Wasser 32 %

Der fein gemahlene Wirkstoff wird mit den Zusatzstoffen innig vermischt. Man erhält so ein

Suspensions-Konzentrat, aus welchem durch Verdünnen mit Wasser Suspensionen jeder gewünschten Konzentration hergestellt werden können.

Beispiel 3: Biologische Prüfungen

3.1. Wirkung gegen Musca domestica

Ein Zuckerwürfel wird derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25°C und 50 % Luftfeuchtigkeit belassen. Nach 24 Stunden wird die insektizide Wirkung durch Ermittlung der Abtötungsrate festgestellt.

Verbindungen gemäss den Beispielen 1.2. zeigen gute Wirkung im obigen Test.

3.2. Kontaktwirkung auf Aphis craccivora

In Töpfen angezogene 4-5 Tage alte Erbsenkeimlinge (Pisum sativum) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe direkt besprüht. Man verwendet pro Test-Verbindung zwei Pflanzen. Eine Auswertung der erzielten Abtötungsrate erfolgt nach 3 und 5 Tagen. Der Versuch wird bei ca. 21°C und einer relativen Luftfeuchtigkeit von etwa 55 % durchgeführt.

Verbindungen gemäss den Beispielen 1.2. zeigen in diesem Test gute Wirkung gegen Aphis craccivora.

3.3. Wirkung gegen ektoparasitäre Zecken

10 frische, vollgesogene Boophilus microplus Weibchen werden in einer Reihe dorsal auf einer PVC Platte aufgeklebt und mit einem Wattebausch überdeckt. Danach werden die Testtiere mit 10 ml der wässrigen Testlösung übergossen. Eine Stunde später wird der Wattebausch entfernt und die Zecken über Nacht bei 24°C getrocknet. Nach dem Trocknen werden die Zecken 4 Wochen lang bis zum Ende der Eiablage und Beginn des Larvenschlupfes bei 28°C und 80 % Luftfeuchtigkeit gehalten.

Jede Testsubstanz wird in einer Konzentration von 500 ppm getestet. Die akarizide Wirkung zeigt sich entweder beim Weibchen als Mortalität oder Sterilität oder im Eigelege durch Blockieren der Embryogenese oder des Schlüpfaktes. Alle Substanzen werden gegen zwei verschiedene Zeckenstämme, den OP-resistenten Stamm BIARRA und den Amidin-resistenten Stamm ULAM geprüft.

Verbindungen gemäss den Beispielen 1.2. zeigen gute Wirkung im obigen Test.

3.4. Wirkung gegen Bodeninsekten (Diabrotica balteata)

Es werden 350 ml Erde (bestehend aus 95 Vol.-% Sand und 5 Vol.-% Torf) mit jeweils 150 ml von wässrigen Emulsionszubereitungen vermischt, die den zu prüfenden Wirkstoff in einer Konzentration von 400 ppm enthalten. Dann werden Plastikbecher, die einen oberen Durchmeser von ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 24°C und einer relativen Luftfeuchtigkeit von ca. 50% gehalten. Sechs Tage nach dem Ansatz wird die in den Bechern enthaltene Erde abgesiebt und die zurückbleibenden Larven werden auf ihre Mortalität geprüft.

Verbindungen gemäss den Beispielen 1.2. zeigen gute Wirkung im obigen Test.

3.5. Frassgift-Wirkung auf Spodoptera littoralis-Larven ($L_1$)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26°C und ca. 50 % relativer Luftfeuchtigkeit durchgeführt. Nach 2 und 3 Tagen wird die Mortalität und nach 5 Tagen werden Entwicklungs- und Häutungsstörungen der angesetzten Larven bestimmt.

Verbindungen gemäss den Beispiel 1.2. zeigen gute Wirkung in diesem Test.

3.6. Frassgift-Wirkung auf Spodoptera littoralis- und Heliothis virescens-Larven ($L_3$)

Eingetopfte Sojapflanzen (Topfgrösse 10 cm Durchmesser) im 4-Blatt-Stadium werden mit wässrigen

Wirkstoffemulsionen besprüht, die den Wirkstoff in einer Konzentration von 400 ppm enthalten.

Nach zwei Tagen werden die behandelten Sojapflanzen mit je 10 Larven von Spodoptera littoralis und Heliothis virescens im dritten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2. zeigen 80-100%ige Wirkung (Mortalität).

3.7. Frassgift-Wirkung auf Plutella xylostella-Larven ($L_2$)

Eingetopfte Chinakohlpflanzen (Topfgrösse 10 cm Durchmesser) im 4-Blatt-Stadium werden mit wässrigen Wirkstoffemulsionen besprüht, die den Wirkstoff in einer Konzentration von 400 ppm enthalten.

Nach zwei Tagen werden die behandelten Chinakohlpflanzen mit 10 Plutella xylostella-Larven im zweiten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2. zeigen 80-100%ige Wirkung (Mortalität).

3.8. Kontaktwirkung gegen Nilaparvata lugens (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 40 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 6 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Beleuchtungsperiode von 16 Stunden durchgeführt.

Verbindungen gemäss den Beispielen 1.2. zeigen in diesem Test gute Wirkung.

3.9. Systemische Wirkung auf Nilaparvata lugens

Etwa 10 Tage alte Reispflanzen (ca. 10 cm hoch) werden in einen Plastik-Becher eingestellt, der 200 ml einer wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wird mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im $N_2$-bis $N_3$- Stadium besiedelt und mit einem Plastikzylinder abgedeckt. Der Versuch wird bei 26°C und ca. 60 % relativer Luftfeuchtigkeit mit einer Beleuchtungsperiode von 16 Stunden durchgeführt. Nach zwei und fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen gemäss den Beispielen 1.2. zeigen im obigen Test 80 - 100%ige Wirkung (Mortalität) gegen Nilaparvata lugens.

3.10. Wirkung gegen pflanzenschädigende Akarinen: Tetranychus urticae (OP-sensible) und Tetranychus cinnabarinus (OP-tolerant)

Die Primärblätter von Phaseolus vulgaris-Pflanzen werden 16 Stunden vor dem Versuch auf akarizide Wirkung mit einem infestierten Blattstück aus einer Massenzucht von Tetranychus urticae (OP-sens.) oder Tetranchus cinnabarinus (OP-tol.) belegt (die Toleranz bezieht sich auf die Verträglichkeit gegenüber Diazinon).

Die so behandelten infestierten Pflanzen werden mit einer Versuchslösung enthaltend 400 ppm der jeweils zu prüfenden Verbindung bis zur Tropfnässe besprüht. Nach 24 Stunden und wiederum nach 7 Tagen werden Imagines und Larven (alle beweglichen Stadien) unter dem Binokular auf lebende und tote Individuen ausgewertet. Man verwendet pro Testspezies eine Pflanze. Während des Versuchsverlaufs stehen die Pflanzen in Gewächshauskabinen bei 25°C.

Verbindungen gemäss den Beispielen 1.2. zeigen in diesem Versuch gute Wirkung gegen Tetranychus urticae und Tetranychus cinnabarinus.

3.11. Ovizide Wirkung auf Tetranychus urticae (OP-resistent)

Eingetopfte Phaseolus vulgaris-Pflanzen im Primärblatt-Stadium werden jeweils zweimal mit 30 Weibchen von Tetranychus urticae besiedelt. Nach 24stündiger Eiablage werden die Weibchen mit einer Saugpumpe (Wasserstrahlpumpe) von den Pflanzen entfernt, so dass auf diesen nur noch die abgelegten Milbeneier

verbleiben.

Die mit den Milbeneiern infestierten Pflanzen werden dann mit einer wässrigen Emulsion enthaltend 400 ppm der zu prüfenden Verbindung bis zur Tropfnässe besprüht und 5 Tage bei 25° C und etwa 50 % relativer Luftfeuchtigkeit gehalten. Nach dieser Zeit wird die prozentuale Mortalität der Eier und geschlüpften Larven durch Auszählen bestimmt.

Verbindungen gemäss den Beispielen 1.2. zeigen gute Wirkung in obigem Test.

3.12. Pflanzenmitizide Blattpenetrations-Wirkung auf Tetranychus cinnabarinus

Für den Versuch werden eingetopfte Buschbohnenpflanzen im Primärblatt-Stadium, die mit Tetranychus cinnabarinus infestiert sind, verwendet. Die Besiedlung mit den Milben erfolgt 1 Tag vor der Wirkstoffapplikation.

Die Blattoberseiten der mit diesen Milben infestierten Versuchspflanzen werden mit einer Emulsionszubereitung enthaltend 400 ppm des zu prüfenden Wirkstoffes besprüht. Nach dem Antrocknen des Sprühbelages wird jeweils der Rand der Blattoberseite einer Anzahl befallener Blätter mit einem Wulst aus zähflüssigem Leim (Raupenleim) abgegrenzt, um ein Ueberlaufen der Milben von der Blattunterseite auf die Blattoberseite zu verhindern.

Die behandelten Pflanzen werden dann im Gewächshaus bei einer Temperatur von 25° C bis 27° C und einer relativen Luftfeuchtigkeit von ca. 50 % gehalten. Sechs Tage nach der Wirkstoffapplikation wird festgestellt, ob eine translaminare Wirkung, d.h. Blattpenetration des Wirkstoffs von der Blattoberseite zur Blattunterseite, eingetreten war, und zwar durch Bestimmung der prozentualen Mortalität der Eier und larvalen sowie adulten Stadien.

Verbindungen gemäss den Beispielen 1.2. zeigen gute Wirkung in obigem Test.

3.13. Wirkung gegen Panonychus ulmi (OP und Carb. resistent)

Eingetopfte Apfelsämlinge mit etwa 20-30 Blättern werden mit jeweils 60 adulten Weibchen von Panonychus ulmi besiedelt. Nach sieben Tagen werden die infestierten Pflanzen mit einer wässrigen Emulsion enthaltend 400 ppm der zu prüfenden Verbindung bis zur Tropfnässe besprüht. Die behandelten Pflanzen werden dann während weiterer 14 Tage bei 25° C und etwa 50 % relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Verbindungen gemäss den Beispielen 1.2. zeigen gute Wirkung in obigem Test.

3.14. Wirkung gegen tierparasitäre Milben

Von mit Dermanyssus gallinae befallenen Hühnern werden Ansätze bestehend aus etwa 50 Milben verschiedener Stadien (Larven, Nymphen und Imagines) entnommen. Die Milbenansätze werden jeweils mit einer wässrigen Emulsion, Suspension bzw. Lösung enthaltend 800 ppm des zu prüfenden Wirkstoffes benetzt. Hierzu werden die Milbenansätze in einem Teströhrchen mit der den Wirkstoff enthaltenden flüssigen Zubereitung übergossen; die Flüssigkeit wird anschliessend mit Hilfe eines Wattebausches aufgesogen. Die so behandelten Milben verbleiben während 72 Stunden in dem Teströhrchen. Nach dieser Zeit wird die prozentuale Abtötung der behandelten Milben gegenüber unbehandelten Kontrollansätzen ermittelt.

Verbindungen gemäss den Beispielen 1.2. zeigen gute Wirkung im obigen Test.

3.15. Wirkung gegen Zecken: Eiablagehemmung

Als Testtiere werden vollgesogene adulte Weibchen der Rinderzecke Boophilus microplus verwendet. Es werden je 10 Zecken eines OP-resistenten Stammes (z.B. vom Biarra-Stamm) und eines normal empfindlichen Stammes (z.B. vom Yeerongpilly-Stamm) behandelt. Die Zecken werden auf mit Doppelklebeband bezogenen Platten fixiert und dann entweder mit wässrigen Emulsionen bzw. mit Lösungen enthaltend 800 ppm der zu untersuchenden Verbindung oder eines ihrer Salze benetzt oder mit einem mit diesen Flüssigkeiten getränkten Wattebausch in Berührung gebracht. Anschliessend werden sie in einem klimatisierten Raum bei konstanten Bedingungen aufbewahrt. Die Auswertung erfolgt nach drei Wochen. Es wird die prozentuale Hemmung der Ablage von fertilen Eiern gegenüber unbehandelten Kontrollen ermittelt.

Verbindungen gemäss den Beispielen 1.2. zeigen gute Wirkung im obigen Test.

**Patentansprüche**

1. Verbindungen der Formel I

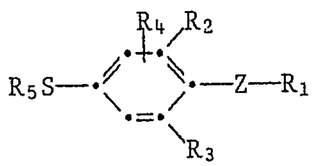 (I),

worin $R_1$ für $C_1$-$C_{10}$-Alkyl, ein- oder mehrfach durch Halogen und/oder $C_1$-$C_6$-Alkoxy substituiertes $C_1$-$C_{10}$-Alkyl, $C_3$-$C_6$-Alkenyl, $C_3$-$C_8$-Cycloalkyl, ein- oder mehrfach durch Halogen und/oder $C_1$-$C_4$-Alkyl substituiertes $C_3$-$C_8$-Cycloalkyl, $C_3$-$C_8$-Cycloalkyl-$C_1$-$C_4$-alkyl, ($C_3$-$C_8$-Cycloalkyl)$_2$-$C_1$-$C_4$-alkyl, Phenyl-$C_1$-$C_4$-alkyl, ein- oder mehrfach im Phenylteil durch Halogen, $C_1$-$C_4$-Alkyl, $C_1$-$C_4$-Alkoxy und/oder Halogen-$C_1$-$C_4$-alkyl substituiertes Phenyl-$C_1$-$C_4$-alkyl oder $C_5$-$C_6$-Cycloalkenyl;
$R_2$ für Wasserstoff, $C_1$-$C_5$-Alkyl oder $C_5$-$C_6$-Cycloalkyl;
$R_3$ für $C_1$-$C_5$-Alkyl oder $C_5$-$C_6$-Cycloalkyl;
$R_4$ für Wasserstoff oder $C_1$-$C_3$-Alkyl;
$R_5$ für einen fünfgliedrigen Heterocyclus mit 1 bis 4 gleichen oder verschiedenen Heteroatomen der Gruppe N, O und S oder einen ein- oder mehrfach durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio und/oder gegebenenfalls substituiertes Phenyl substituierten Heterocyclus mit 1 bis 4 gleichen oder verschiedenen Heteroatomen der Gruppe N, O und S;
Z für -NH-CS-NH-, -N=C($SR_6$)-NH- oder -N=C=N-; und
$R_6$ für $C_1$-$C_5$-Alkyl, $C_2$-$C_5$-Alkenyl, $C_3$-$C_4$-Alkinyl, $C_1$-$C_4$-Alkoxy-$C_1$-$C_4$-alkyl oder $C_1$-$C_4$-Alkylthio-$C_1$-$C_4$-alkyl stehen, sowie ihre Salze mit organischen oder anorganischen Säuren.

2. Verbindungen der Formel I gemäss Anspruch 1, worin $R_1$ für $C_1$-$C_7$-Alkyl, ein- oder mehrfach durch Halogen und/oder $C_1$-$C_5$-Alkoxy substituiertes $C_1$-$C_7$-Alkyl, $C_5$-$C_6$-Cycloalkyl, $C_3$-$C_5$-Cycloalkyl-$C_1$-$C_3$-alkyl oder ($C_3$-$C_5$-Cycloalkyl)$_2$-$C_1$-$C_3$-alkyl; $R_2$ für $C_1$-$C_4$-Alkyl oder $C_5$-$C_6$-Cycloalkyl; $R_3$ für $C_1$-$C_4$-Alkyl; $R_4$ für Wasserstoff; $R_5$ für

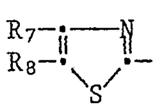 , 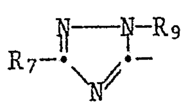 , 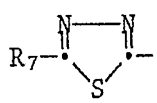 oder 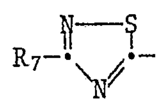 ,

wobei $R_7$ und $R_8$ Wasserstoff, Chlor, Brom, $C_1$-$C_4$-Alkyl oder Halogen-$C_1$-$C_4$-alkyl und $R_9$ $C_1$-$C_5$-Alkyl bedeuten; Z für -NH-CS-NH-, -N=C($SR_6$)-NH- oder -N=C=N-; und $R_6$ für $C_1$-$C_5$-Alkyl stehen.

3. Verbindungen der Formel I gemäss Anspruch 2, worin $R_1$ für $C_3$-$C_5$-Alkyl oder $C_5$-$C_6$-Cycloalkyl; $R_2$ und $R_3$ je für $C_1$-$C_4$-Alkyl; $R_4$ für Wasserstoff; $R_5$ für

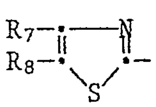 oder 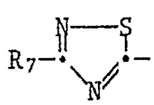 ,

wobei $R_7$ und $R_8$ je Chlor, Brom oder Methyl bedeuten; und Z für -NH-CS-NH- stehen.

4. Verbindungen der Formel I gemäss Anspruch 2, worin $R_1$ für $C_3$-$C_5$-Alkyl oder $C_5$-$C_6$-Cycloalkyl; $R_2$ und $R_3$ je für $C_1$-$C_4$-Alkyl; $R_4$ für Wasserstoff; $R_5$ für

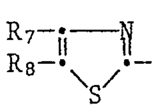 oder 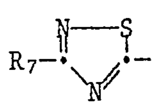 ,

wobei $R_7$ und $R_8$ je Chlor, Brom oder Methyl bedeuten; Z für -N=C($SR_6$)-NH-; und $R_6$ für Methyl oder Aethyl stehen.

5. Verbindungen der Formel I gemäss Anspruch 2, worin $R_1$ für $C_3$-$C_5$-Alkyl oder $C_5$-$C_6$-Cycloalkyl; $R_2$ und $R_3$ je für $C_1$-$C_4$-Alkyl; $R_4$ für Wasserstoff; $R_5$ für

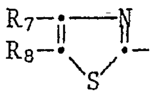 oder 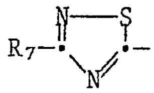 ,

wobei $R_7$ und $R_8$ je Chlor, Brom oder Methyl bedeuten; und Z für -N=C=N- stehen.

6. Die Verbindungen gemäss Anspruch 1 und 3 der Formeln

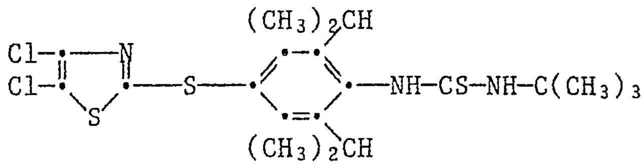 ,

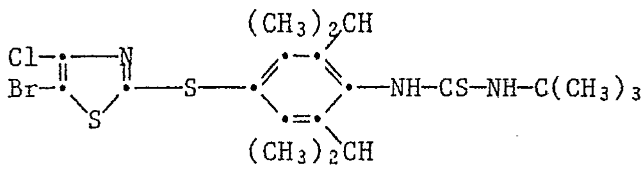 ,

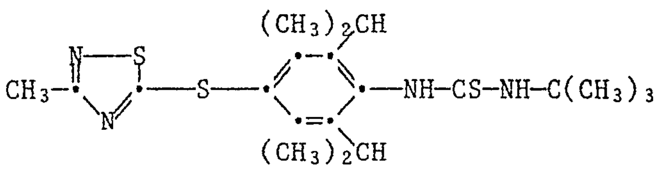 ,

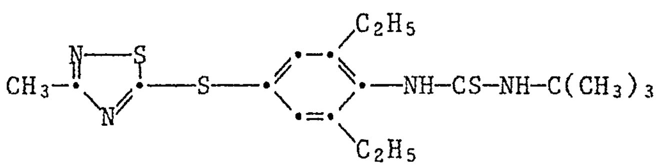 ,

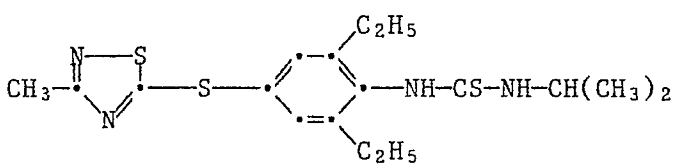 ,

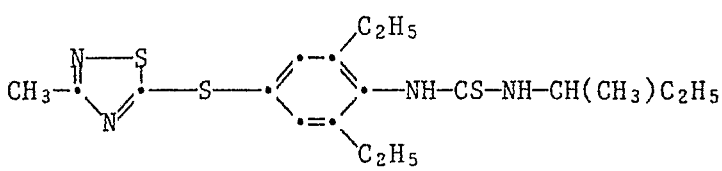 ,

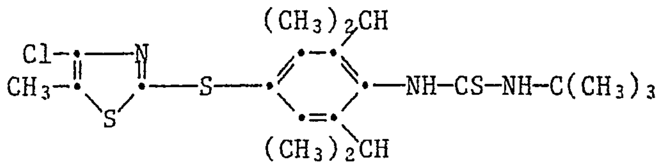
,

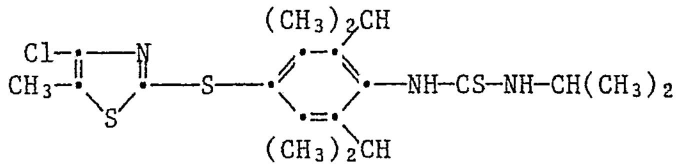
,

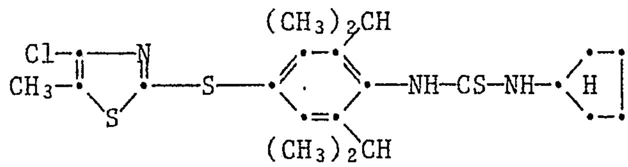
,

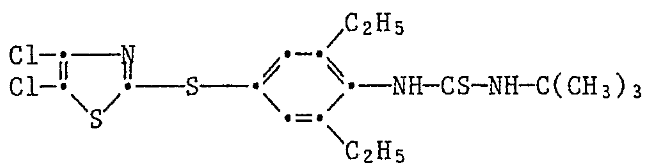
,

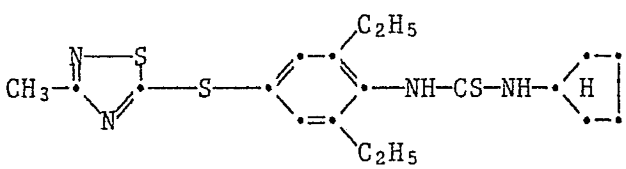
,

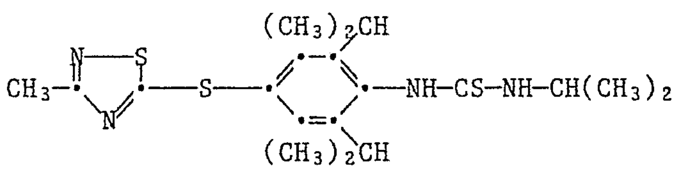
,

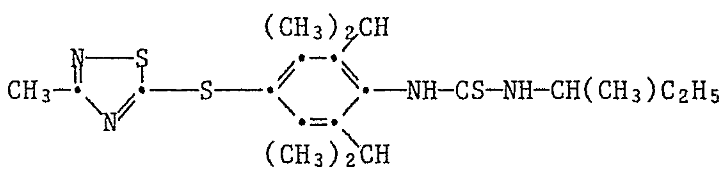
,

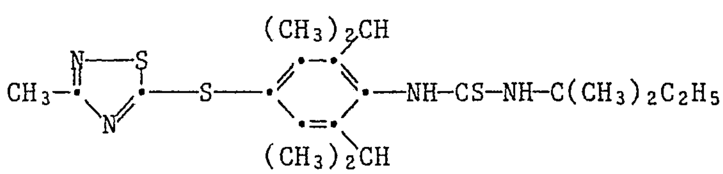
,

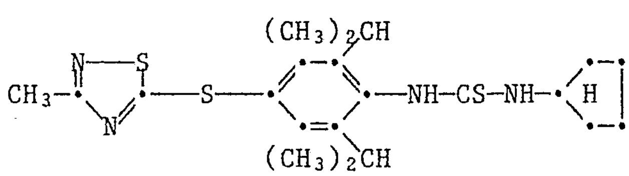
,

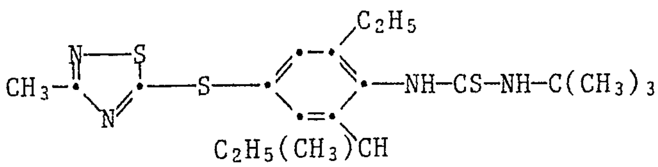 ,

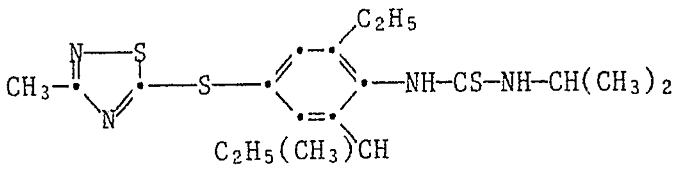 ,

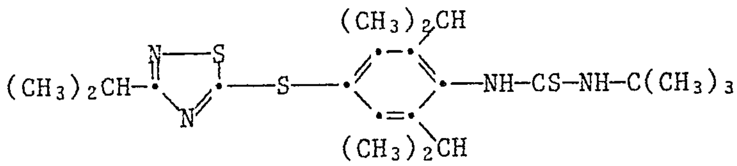 ,

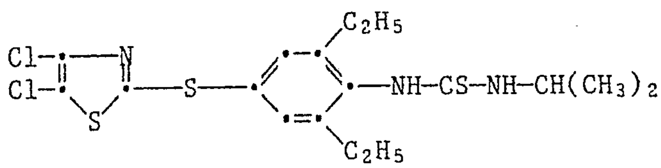 ,

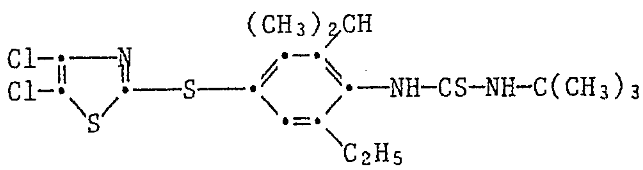 ,

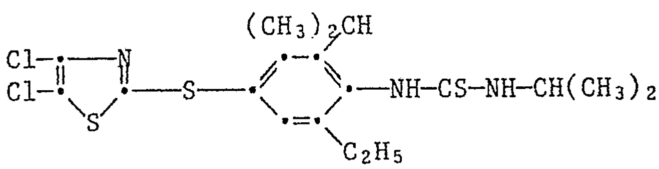 ,

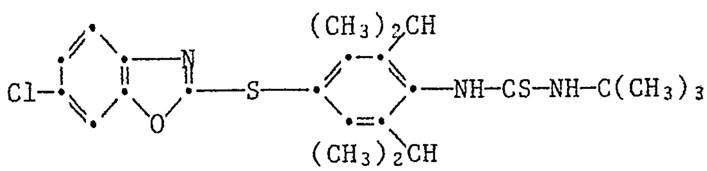 ,

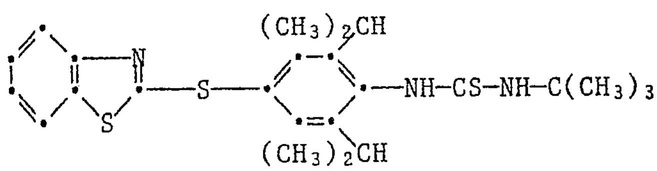 ,

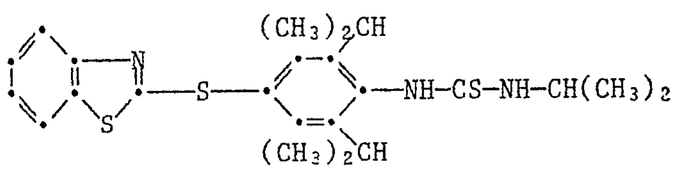 ,

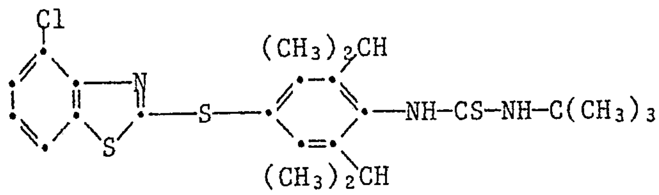
,

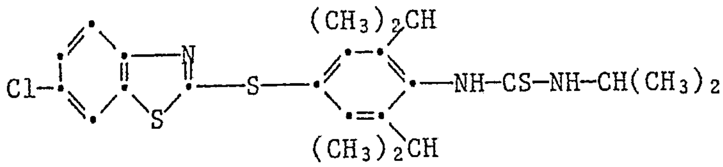
und

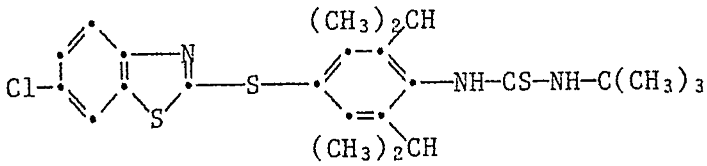
.

7. Die Verbindungen gemäss Anspruch 1 und 4 der Formeln

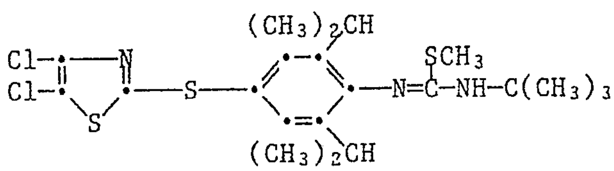
,

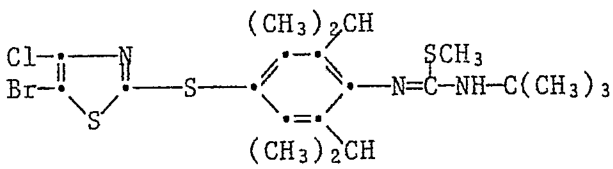
,

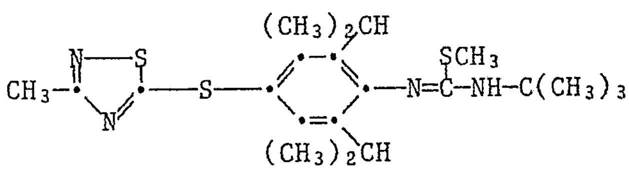
,

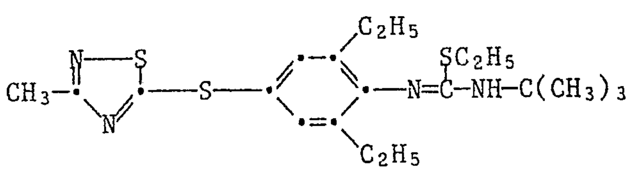
,

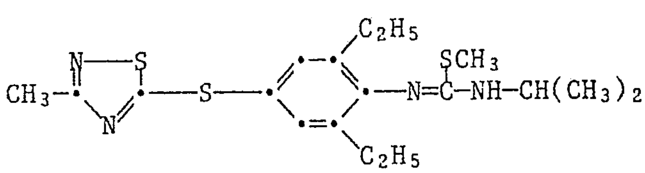
,

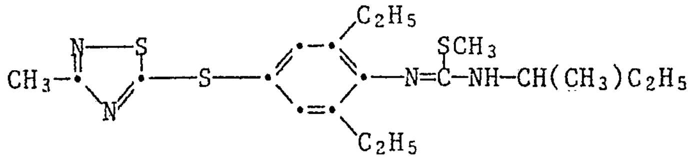

,

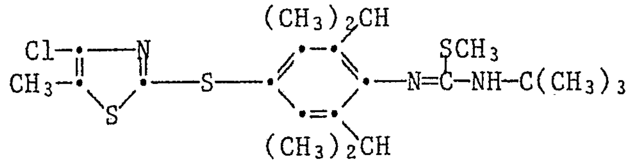

,

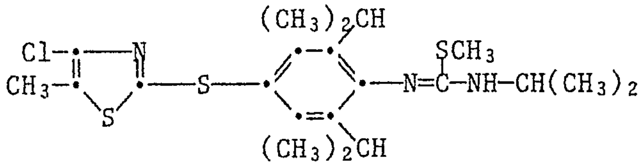

,

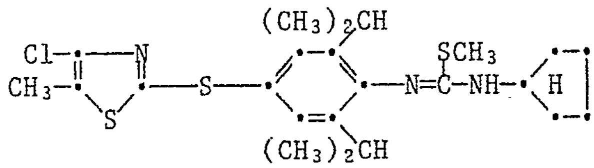

,

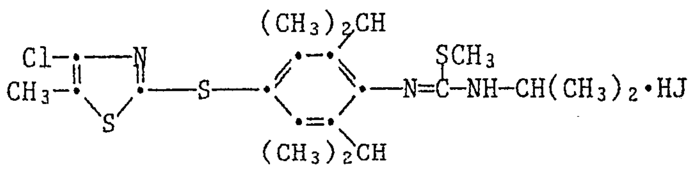

,

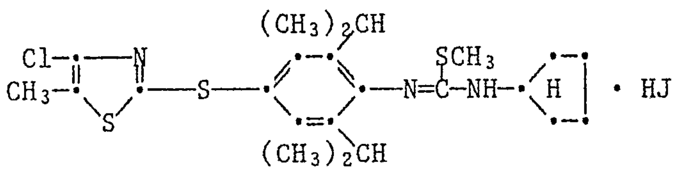

,

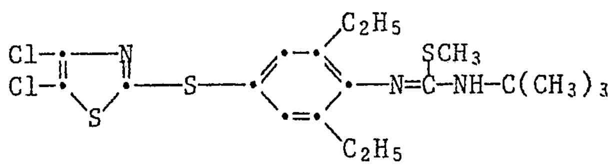

,

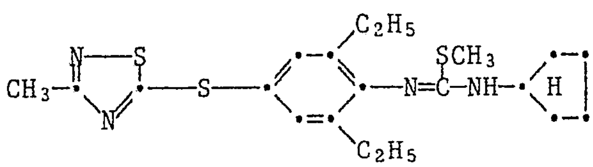

,

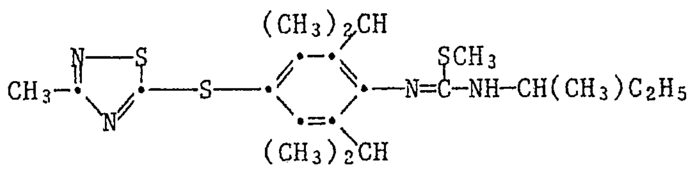

,

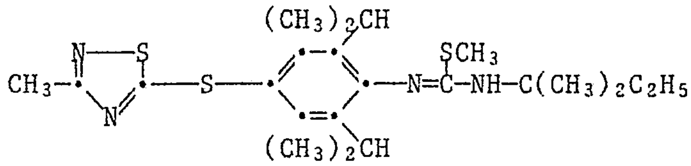 ,

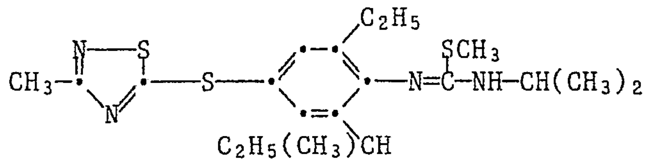 ,

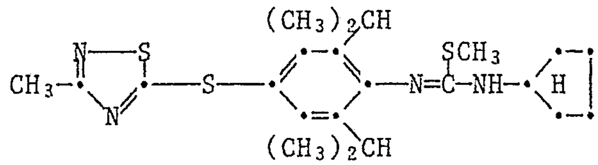 ,

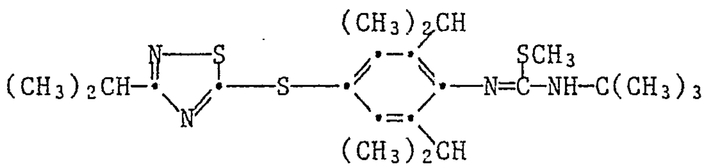 ,

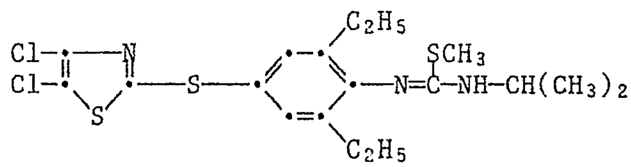 ,

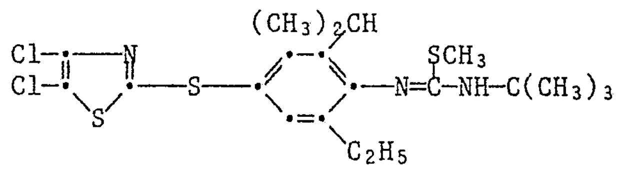 ,

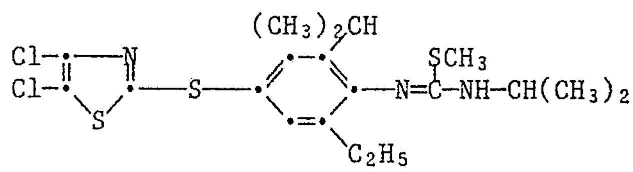 ,

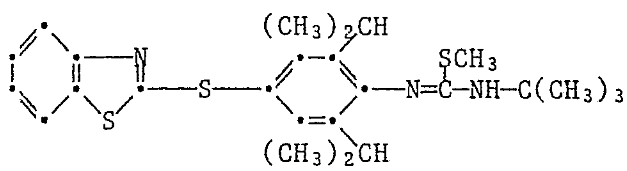 ,

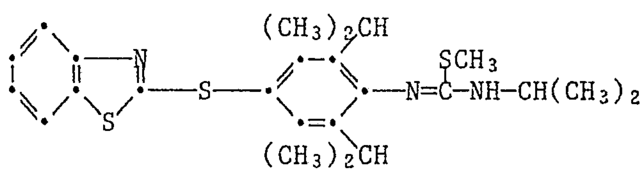 ,

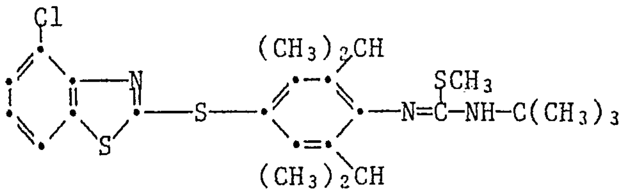

,

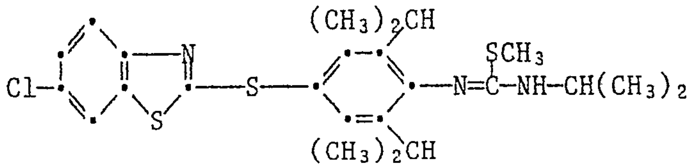

,

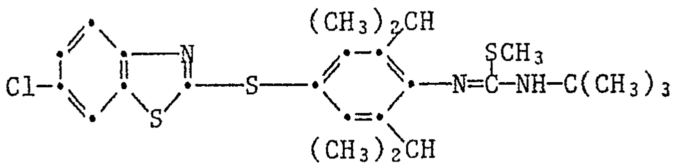

,

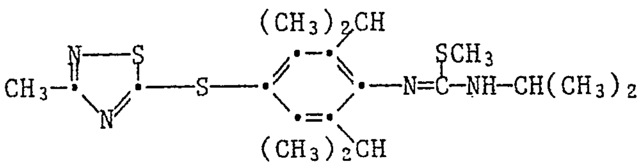

,

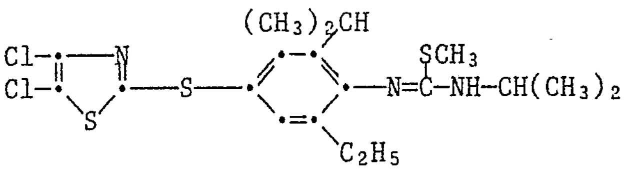

• HJ ,

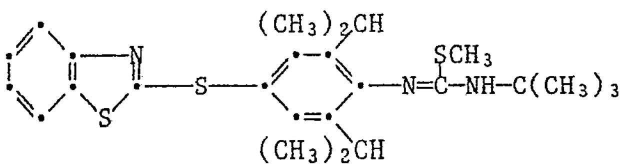

• HJ ,

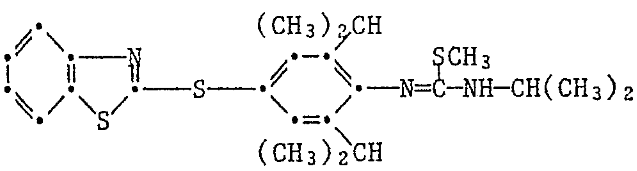

• HJ und

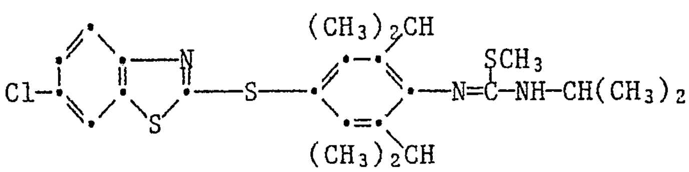

• HJ .

8. Die Verbindungen gemäss Anspruch 1 und 5 der Formeln

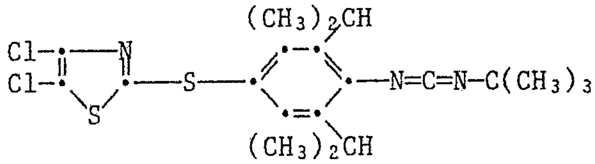
,

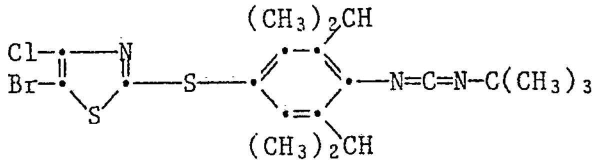
,

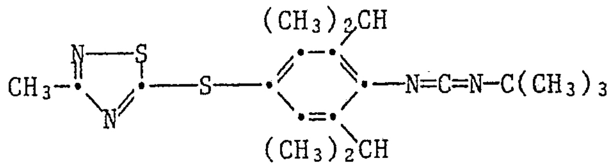
,

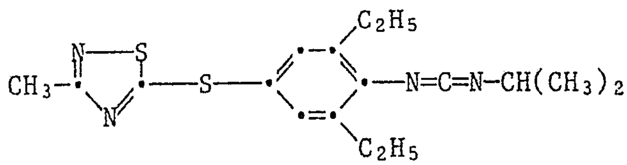
,

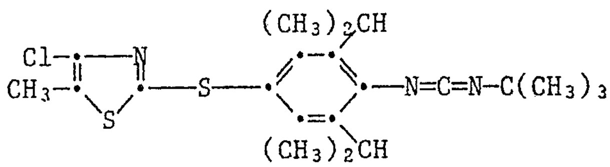
,

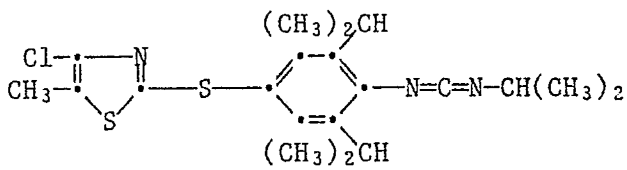
,

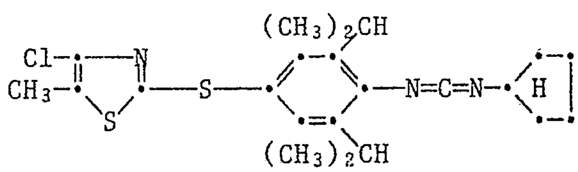
,

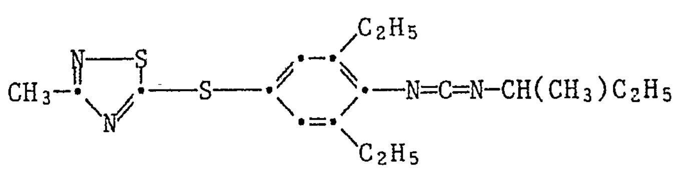
,

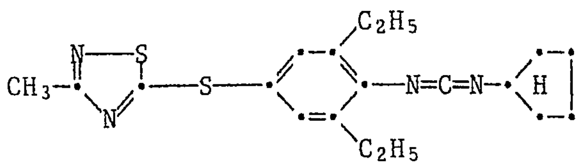

,

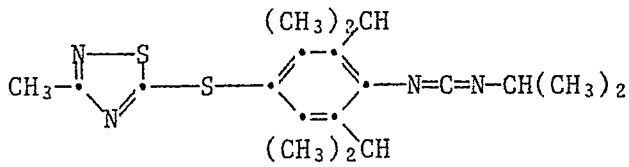

,

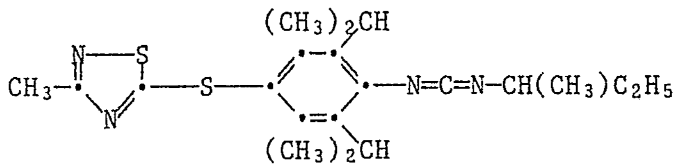

,

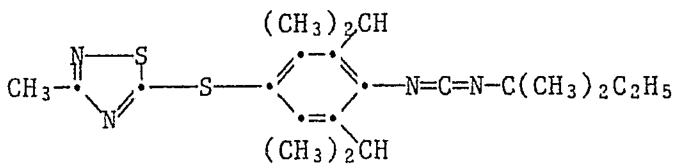

,

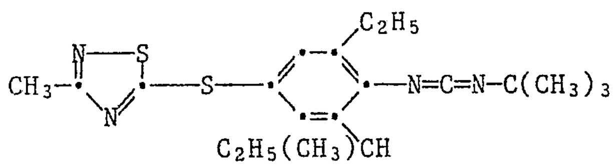

,

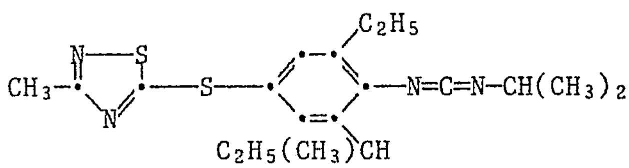

,

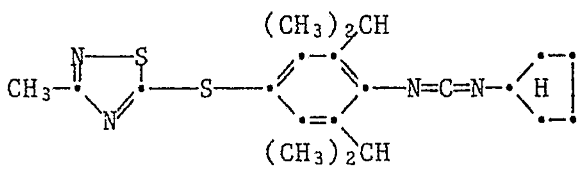

,

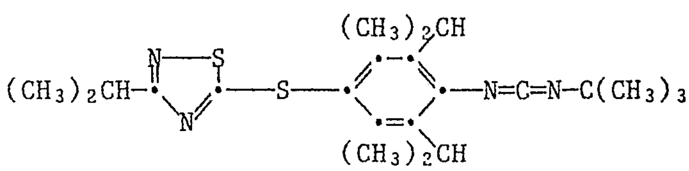

,

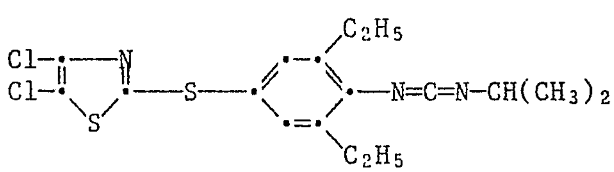

,

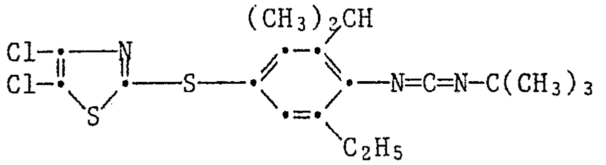

,

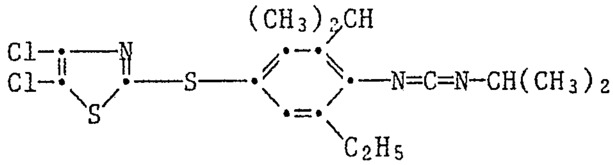

,

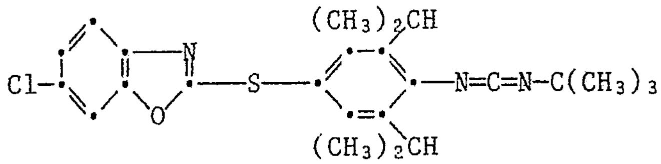

,

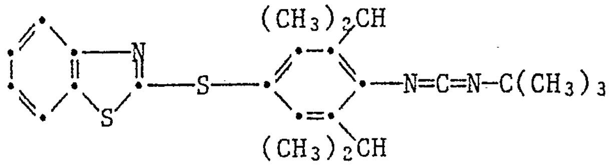

,

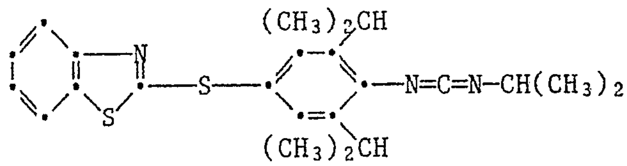

,

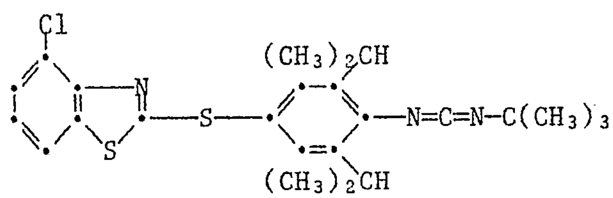

,

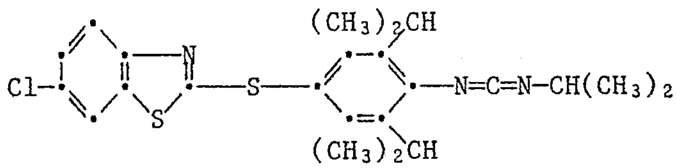

und

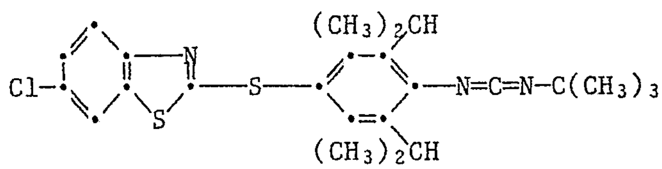

.

9. Verfahren zur Herstellung einer Verbindung der Formel I

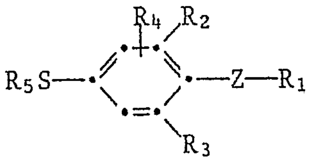

(I),

worin $R_1$ für $C_1$-$C_{10}$-Alkyl, ein- oder mehrfach durch Halogen und/oder $C_1$-$C_6$-Alkoxy substituiertes $C_1$-$C_{10}$-Alkyl, $C_3$-$C_6$-Alkenyl, $C_3$-$C_8$-Cycloalkyl, ein- oder mehrfach durch Halogen und/oder $C_1$-$C_4$-Alkyl substituiertes $C_3$-$C_8$-Cycloalkyl, $C_3$-$C_8$-Cycloalkyl-$C_1$-$C_4$-alkyl, ($C_3$-$C_8$-Cycloalkyl)$_2$-$C_1$-$C_4$-alkyl, Phenyl-$C_1$-$C_4$-alkyl, ein- oder mehrfach im Phenylteil durch Halogen, $C_1$-$C_4$-Alkyl, $C_1$-$C_4$-Alkoxy und/oder Halogen-$C_1$-$C_4$-alkyl substituiertes Phenyl-$C_1$-$C_4$-alkyl oder $C_5$-$C_6$-Cycloalkenyl;
$R_2$ für Wasserstoff, $C_1$-$C_5$-Alkyl oder $C_5$-$C_6$-Cycloalkyl;
$R_3$ für $C_1$-$C_5$-Alkyl oder $C_5$-$C_6$-Cycloalkyl;
$R_4$ für Wasserstoff oder $C_1$-$C_3$-Alkyl;
$R_5$ für einen fünfgliedrigen Heterocyclus mit 1 bis 4 gleichen oder verschiedenen Heteroatomen der Gruppe N, O und S oder einen ein- oder mehrfach durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio und/oder gegebenenfalls substituiertes Phenyl substituierten Heterocyclus mit 1 bis 4 gleichen oder verschiedenen Heteroatomen der Gruppe N, O und S;
Z für -NH-CS-NH-, -N=C($SR_6$)-NH- oder -N=C=N-; und
$R_6$ für $C_1$-$C_5$-Alkyl, $C_2$-$C_5$-Alkenyl, $C_3$-$C_4$-Alkinyl, $C_1$-$C_4$-Alkoxy-$C_1$-$C_4$-alkyl oder $C_1$-$C_4$-Alkylthio-$C_1$-$C_4$-alkyl stehen, dadurch gekennzeichnet, dass man

A) ein Isothiocyanat der Formel II

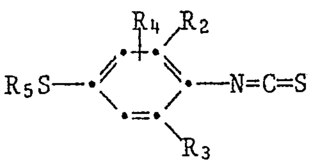

(II)

mit einem Amin der Formel III

$H_2N-R_1$ (III)

in einem organischen Lösungs- oder Verdünnungsmittel bei Normaldruck und einer Temperatur von 0 bis +150° C zum Thioharnstoff umsetzt und gegebenenfalls

B) den erhaltenen Thioharnstoff mit einer Verbindung der Formel IV

$X-R_6$ (IV)

in einem inerten organischen Lösungsmittel unter leicht erhöhtem oder normalem Druck und bei einer Temperatur von +10 bis 250° C zum Isothioharnstoff umsetzt oder

C) den erhaltenen Thioharnstoff durch Abspalten von Schwefelwasserstoff in einem aprotischen organischen Lösungs- oder Verdünnungsmittel unter Normaldruck und bei einer Temperatur von 0 bis +150° C in das Carbodiimid überführt. $R_1$, $R_2$, $R_3$, $R_4$, $R_5$, und $R_6$ die angegebene Bedeutung haben und X steht für eine Abgangsgruppe steht.

10. Schädlingsbekämpfungsmittel, welches als aktive Komponente mindestens eine Verbindung der Formel I

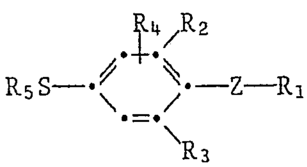

(I)

enthält,
worin $R_1$ für $C_1$-$C_{10}$-Alkyl, ein- oder mehrfach durch Halogen und/oder $C_1$-$C_6$-Alkoxy substituiertes $C_1$-$C_{10}$-Alkyl, $C_3$-$C_6$-Alkenyl, $C_3$-$C_8$-Cycloalkyl, ein- oder mehrfach durch Halogen und/oder $C_1$-$C_4$-Alkyl

substituiertes $C_3$-$C_8$-Cycloalkyl, $C_3$-$C_8$-Cyclo alkyl-$C_1$-$C_4$-alkyl, ($C_3$-$C_8$-Cycloalkyl)$_2$-$C_1$-$C_4$-alkyl, Phenyl-$C_1$-$C_4$-alkyl, ein- oder mehrfach im Phenylteil durch Halogen, $C_1$-$C_4$-Alkyl, $C_1$-$C_4$-Alkoxy und/oder Halogen-$C_1$-$C_4$-alkyl substituiertes Phenyl-$C_1$-$C_4$-alkyl oder $C_5$-$C_6$-Cycloalkenyl;
$R_2$ für Wasserstoff, $C_1$-$C_5$-Alkyl oder $C_5$-$C_6$-Cycloalkyl;
$R_3$ für $C_1$-$C_5$-Alkyl oder $C_5$-$C_6$-Cycloalkyl;
$R_4$ für Wasserstoff oder $C_1$-$C_3$-Alkyl;
$R_5$ für einen fünfgliedrigen Heterocyclus mit 1 bis 4 gleichen oder verschiedenen Heteroatomen der Gruppe N, O und S oder einen ein- oder mehrfach durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio und/oder gegebenenfalls substituiertes Phenyl substituierten Heterocyclus mit 1 bis 4 gleichen oder verschiedenen Heteroatomen der Gruppe N, O und S;
Z für -NH-CS-NH-, -N=C($SR_6$)-NH- oder -N=C=N-; und
$R_6$ für $C_1$-$C_5$-Alkyl, $C_2$-$C_5$-Alkenyl, $C_3$-$C_4$-Alkinyl, $C_1$-$C_4$-Alkoxy-$C_1$-$C_4$-alkyl oder $C_1$-$C_4$-Alkylthio-$C_1$-$C_4$-alkyl stehen, oder eines ihrer Salze mit einer organischen oder
anorganischen Säure zusammen mit geeigneten Trägerstoffen und/oder Zuschlagstoffen.

11. Schädlingsbekämpfungsmittel gemäss Anspruch 10, welches als aktive Komponente mindestens eine Verbindung der Formel I enthält, worin $R_1$ für $C_1$-$C_7$-Alkyl, ein- oder mehrfach durch Halogen und/oder $C_1$-$C_5$-Alkoxy substituiertes $C_1$-$C_7$-Alkyl, $C_5$-$C_6$-Cycloalkyl, $C_3$-$C_5$-Cycloalkyl-$C_1$-$C_3$-alkyl oder ($C_3$-$C_5$-Cycloalkyl)$_2$-$C_1$-$C_3$-alkyl; $R_2$ für $C_1$-$C_4$-Alkyl oder $C_5$-$C_6$-Cycloalkyl; $R_3$ für $C_1$-$C_4$-Alkyl;
$R_4$ für Wasserstoff; $R_5$ für

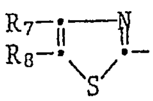 , 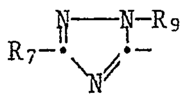 , 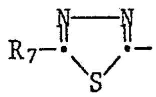 oder 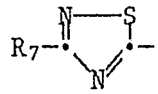 ,

wobei $R_7$ und $R_8$ Wasserstoff, Chlor, Brom, $C_1$-$C_4$-Alkyl oder Halogen-$C_1$-$C_4$-alkyl und $R_9$ $C_1$-$C_5$-Alkyl bedeuten;
Z für -NH-CS-NH-, -N=C($SR_6$)-NH- oder -N=C=N-; und $R_6$ für $C_1$-$C_5$-Alkyl stehen.

12. Schädlingsbekämpfungsmittel gemäss Anspruch 11, worin $R_1$ für $C_3$-$C_5$-Alkyl oder $C_5$-$C_6$-Cycloalkyl; $R_2$ und $R_3$ je für $C_1$-$C_4$-Alkyl; $R_4$ für Wasserstoff; $R_5$ für

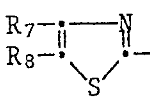 oder 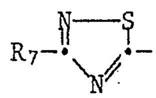 ,

wobei $R_7$ und $R_8$ je Chlor, Brom oder Methyl bedeuten; und Z für -NH-CS-NH- stehen.

13. Schädlingsbekämpfungsmittel gemäss Anspruch 11, worin $R_1$ für $C_3$-$C_5$-Alkyl oder $C_5$-$C_6$-Cycloalkyl; $R_2$ und $R_3$ je für $C_1$-$C_4$-Alkyl; $R_4$ für Wasserstoff; $R_5$ für

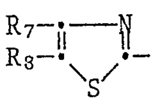 oder 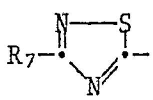 ,

wobei $R_7$ und $R_8$ je Chlor, Brom oder Methyl bedeuten; Z für -N=C($SR_6$)-NH-; und $R_6$ für Methyl oder Aethyl stehen.

14. Schädlingsbekämpfungsmittel gemäss Anspruch 11, worin $R_1$ für $C_3$-$C_5$-Alkyl oder $C_5$-$C_6$-Cycloalkyl; $R_2$ und $R_3$ je für $C_1$-$C_4$-Alkyl; $R_4$ für Wasserstoff; $R_5$ für

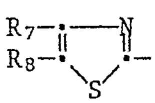 oder 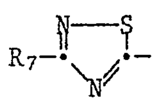 ,

wobei $R_7$ und $R_8$ je Chlor, Brom oder Methyl bedeuten; und Z für -N=C=N- stehen.

15. Verwendung einer Verbindung der Formel I

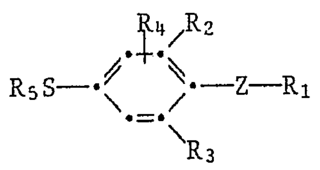

(I)

worin $R_1$ für $C_1$-$C_{10}$-Alkyl, ein- oder mehrfach durch Halogen und/oder $C_1$-$C_6$-Alkoxy substituiertes $C_1$-$C_{10}$-Alkyl, $C_3$-$C_6$-Alkenyl, $C_3$-$C_8$-Cycloalkyl, ein- oder mehrfach durch Halogen und/oder $C_1$-$C_4$-Alkyl substituiertes $C_3$-$C_8$-Cycloalkyl, $C_3$-$C_8$-Cycloalkyl-$C_1$-$C_4$-alkyl, $(C_3$-$C_8$-Cycloalkyl$)_2$-$C_1$-$C_4$-alkyl, Phenyl-$C_1$-$C_4$-alkyl, ein- oder mehrfach im Phenylteil durch Halogen, $C_1$-$C_4$-Alkyl, $C_1$-$C_4$-Alkoxy und/oder Halogen-$C_1$-$C_4$-alkyl substituiertes Phenyl-$C_1$-$C_4$-alkyl oder $C_5$-$C_6$-Cycloalkenyl;
$R_2$ für Wasserstoff, $C_1$-$C_5$-Alkyl oder $C_5$-$C_6$-Cycloalkyl;
$R_3$ für $C_1$-$C_5$-Alkyl oder $C_5$-$C_6$-Cycloalkyl;
$R_4$ für Wasserstoff oder $C_1$-$C_3$-Alkyl;
$R_5$ für einen fünfgliedrigen Heterocyclus mit 1 bis 4 gleichen oder verschiedenen Heteroatomen der Gruppe N, O und S oder einen ein- oder mehrfach durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio und/oder gegebenenfalls substituiertes Phenyl substituierten Heterocyclus mit 1 bis 4 gleichen oder verschiedenen Heteroatomen der Gruppe N, O und S;
Z für -NH-CS-NH-, -N=C($SR_6$)-NH- oder -N=C=N-; und
$R_6$ für $C_1$-$C_5$-Alkyl, $C_2$-$C_5$-Alkenyl, $C_3$-$C_4$-Alkinyl, $C_1$-$C_4$-Alkoxy-$C_1$-$C_4$-alkyl oder $C_1$-$C_4$-Alkylthio-$C_1$-$C_4$-alkyl stehen, oder eines ihrer Salze mit einer organischen oder anorganischen Säure zur Bekämpfung von Schädlingen an Tieren und Pflanzen.

16. Verwendung gemäss Anspruch 15 einer Verbindung der Formel I zur Bekämpfung von Insekten und Arachniden.

17. Verfahren zur Bekämpfung von Schädlingen an Tieren und Pflanzen, dadurch gekennzeichnet, dass man die Schädlinge in ihren verschiedenen Entwicklungsstadien mit einer Verbindung der Formel I

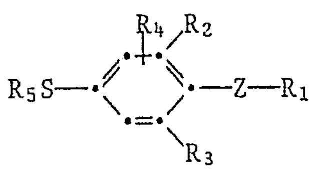

(I)

worin $R_1$ für $C_1$-$C_{10}$-Alkyl, ein- oder mehrfach durch Halogen und/oder $C_1$-$C_6$-Alkoxy substituiertes $C_1$-$C_{10}$-Alkyl, $C_3$-$C_6$-Alkenyl, $C_3$-$C_8$-Cycloalkyl, ein- oder mehrfach durch Halogen und/oder $C_1$-$C_4$-Alkyl substituiertes $C_3$-$C_8$-Cycloalkyl, $C_3$-$C_8$-Cycloalkyl-$C_1$-$C_4$-alkyl, $(C_3$-$C_8$-Cycloalkyl$)_2$-$C_1$-$C_4$-alkyl, Phenyl-$C_1$-$C_4$-alkyl, ein- oder mehrfach im Phenylteil durch Halogen, $C_1$-$C_4$-Alkyl, $C_1$-$C_4$-Alkoxy und/oder Halogen-$C_1$-$C_4$-alkyl substituiertes Phenyl-$C_1$-$C_4$-alkyl oder $C_5$-$C_6$-Cycloalkenyl;
$R_2$ für Wasserstoff, $C_1$-$C_5$-Alkyl oder $C_5$-$C_6$-Cycloalkyl;
$R_3$ für $C_1$-$C_5$-Alkyl oder $C_5$-$C_6$-Cycloalkyl;
$R_4$ für Wasserstoff oder $C_1$-$C_3$-Alkyl;
$R_5$ für einen fünfgliedrigen Heterocyclus mit 1 bis 4 gleichen oder verschiedenen Heteroatomen der Gruppe N, O und S oder einen ein- oder mehrfach durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio und/oder gegebenenfalls substituiertes Phenyl substituierten Heterocyclus mit 1 bis 4 gleichen oder verschiedenen Heteroatomen der Gruppe N, O und S;
Z für -NH-CS-NH-, -N=C($SR_6$)-NH- oder -N=C=N-; und
$R_6$ für $C_1$-$C_5$-Alkyl, $C_2$-$C_5$-Alkenyl, $C_3$-$C_4$-Alkinyl, $C_1$-$C_4$-Alkoxy-$C_1$-$C_4$-alkyl oder $C_1$-$C_4$-Alkylthio-$C_1$-$C_4$-alkyl stehen, oder einem ihrer Salze mit einer organischen oder anorganischen Säure in Kontakt bringt.

18. Verbindungen der Formel I

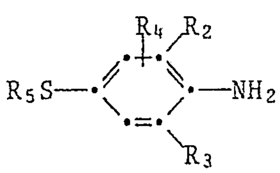

(V)

worin $R_2$ für Wasserstoff, $C_1$-$C_5$-Alkyl oder $C_5$-$C_6$-Cycloalkyl;
$R_3$ für $C_1$-$C_5$-Alkyl oder $C_5$-$C_6$-Cycloalkyl;

$R_4$ für Wasserstoff oder $C_1$-$C_3$-Alkyl; und
$R_5$ für einen fünfgliedrigen Heterocyclus mit 1 bis 4 gleichen oder verschiedenen Heteroatomen der Gruppe N, O und S oder einen ein- oder mehrfach durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio und/oder gegebenenfalls substituiertes Phenyl substituierten Heterocyclus mit 1 bis 4 gleichen oder verschiedenen Heteroatomen der Gruppe N, O und S stehen.

19. Verbindungen der Formel V gemäss Anspruch 18, worin $R_2$ für $C_1$-$C_4$-Alkyl oder $C_5$-$C_6$-Cycloalkyl; $R_3$ für $C_1$-$C_4$-Alkyl; $R_4$ für Wasserstoff; und $R_5$ für

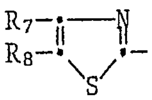 , 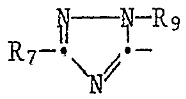 , 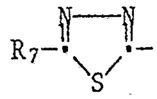 oder 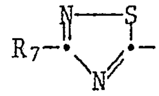 ,

stehen, wobei $R_7$ und $R_8$ Wasserstoff, Chlor, Brom, $C_1$-$C_4$-Alkyl oder Halogen-$C_1$-$C_4$-alkyl und $R_9$ $C_1$-$C_5$-Alkyl bedeuten.

20. Verbindungen der Formel V gemäss Anspruch 19, worin $R_2$ und $R_3$ je für $C_1$-$C_4$-Alkyl; $R_4$ für Wasserstoff; und $R_5$ für

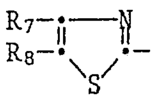 oder 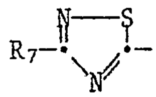 ,

stehen, wobei $R_7$ und $R_8$ je Chlor, Brom oder Methyl bedeuten.

21. Die Verbindungen gemäss Anspruch 18 der Formeln

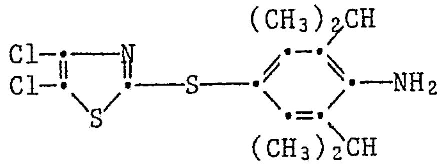
,

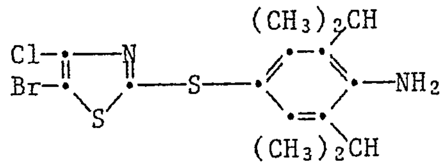
,

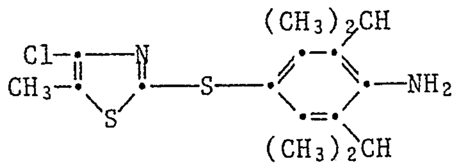
,

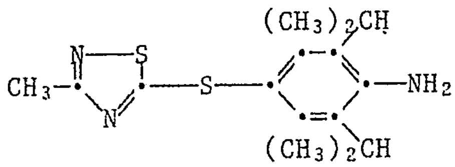
,

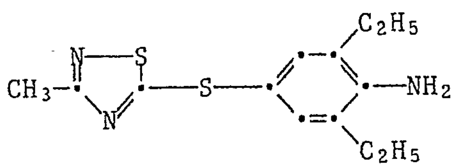
,

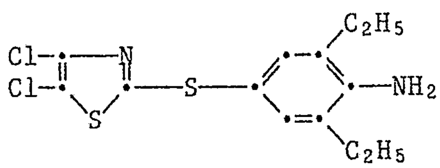
,

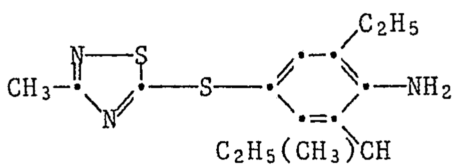
,

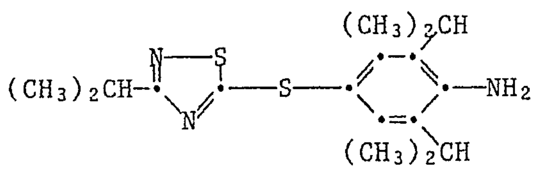
,

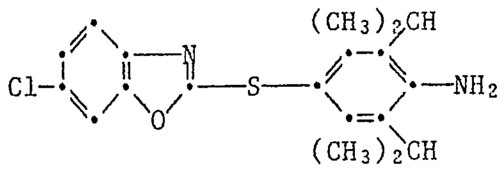 ,

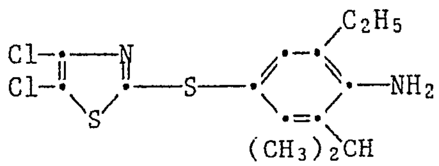 ,

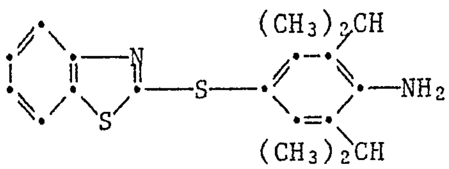 ,

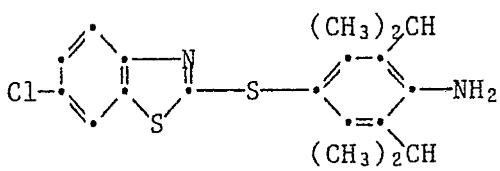 und

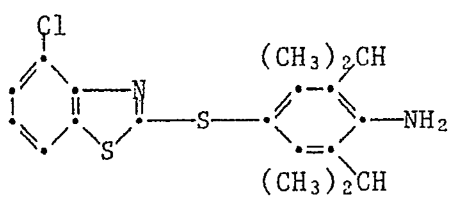 .

22. Verbindungen der Formel II

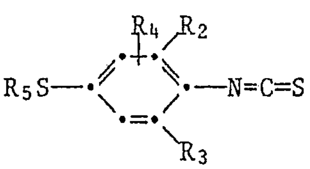 (II),

worin $R_2$ für Wasserstoff, $C_1$-$C_5$-Alkyl oder $C_5$-$C_6$-Cycloalkyl;
$R_3$ für $C_1$-$C_5$-Alkyl oder $C_5$-$C_6$-Cycloalkyl;
$R_4$ für Wasserstoff oder $C_1$-$C_3$-Alkyl;
$R_5$ für einen fünfgliedrigen Heterocyclus mit 1 bis 4 gleichen oder verschiedenen Heteroatomen der Gruppe N, O und S oder einen ein- oder mehrfach durch Halogen, $C_1$-$C_4$-Alkyl, $C_1$-$C_4$-Alkoxy, Halogen-$C_1$-$C_4$-alkyl, Halogen-$C_1$-$C_4$-alkoxy, $C_1$-$C_4$-Alkylthio und/oder gegebenenfalls substituiertes Phenyl substituierten Heterocyclus mit 1 bis 4 gleichen oder verschiedenen Heteroatomen der Gruppe N, O und S stehen.

23. Verbindungen der Formel II gemäss Anspruch 22, worin $R_2$ für $C_1$-$C_4$-Alkyl oder $C_5$-$C_6$-Cycloalkyl ; $R_3$ für $C_1$-$C_4$-Alkyl; $R_4$ für Wasserstoff; und $R_5$ für

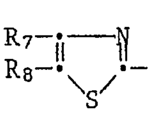 , 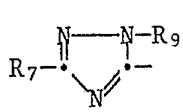 , 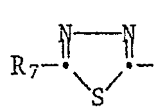 oder 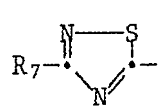 ,

stehen, wobei $R_7$ und $R_8$ Wasserstoff, Chlor, Brom, $C_1$-$C_4$-Alkyl oder Halogen-$C_1$-$C_4$-alkyl und $R_9$ $C_1$-$C_5$-Alkyl bedeuten.

24. Verbindungen der Formel II gemäss Anspruch 23, worin $R_2$ und $R_3$ je für $C_1$-$C_4$-Alkyl; $R_4$ für Wasserstoff; und $R_5$ für

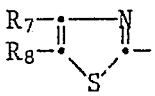

oder

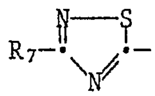

,

stehen, wobei $R_7$ und $R_8$ je Chlor, Brom oder Methyl bedeuten.

25. Die Verbindungen gemäss Anspruch 22 der Formeln

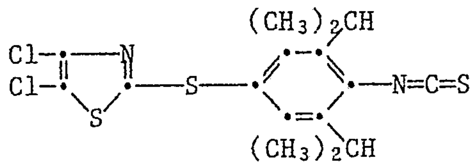

,

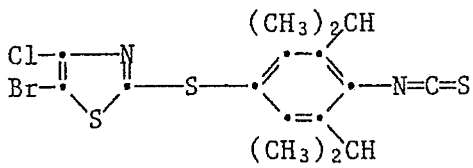

,

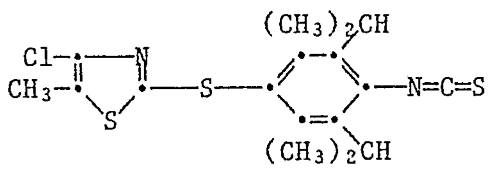

,

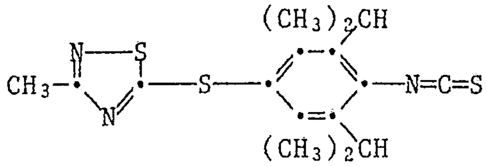 ,

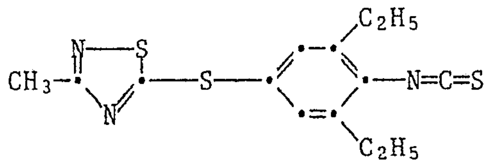 ,

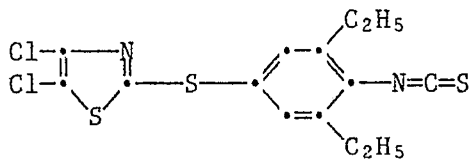 ,

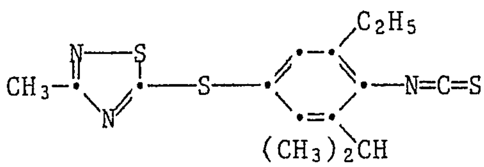 ,

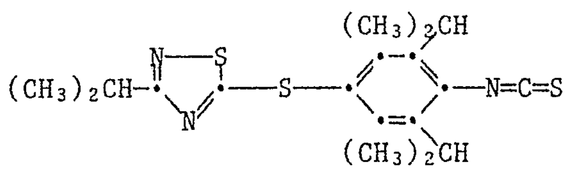 ,

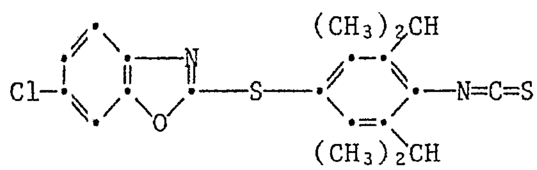 ,

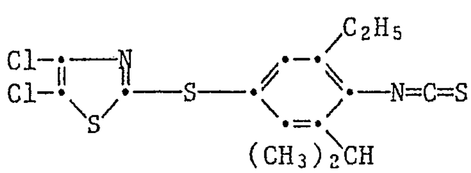 ,

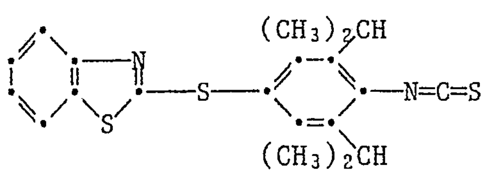 ,

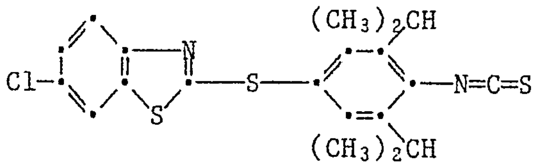

und

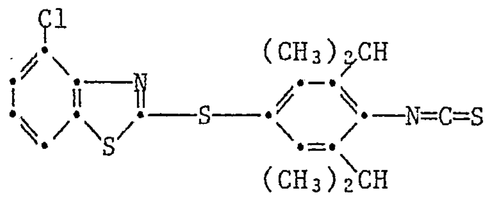

.

FO 7.5/BY/gb*/kg*

Europäisches Patentamt

# EUROPÄISCHER RECHERCHENBERICHT

Nummer der Anmeldung

EP 88 81 0499

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 107 123 (BASF)<br>* Ansprüche *<br>--- | 1,10 | C 07 D 277/36<br>C 07 D 277/74<br>C 07 D 263/58<br>C 07 D 285/08<br>A 01 N 47/30<br>A 01 N 47/00<br>C 07 D 249/12<br>C 07 D 285/12 |
| A | EP-A-0 025 010 (CIBA-GEIGY)<br>* Ansprüche *<br>--- | 1,10 | |
| A | EP-A-0 177 710 (BAYER AG)<br>* Ansprüche *<br>--- | 1,10 | |
| P,X | EP-A-0 262 087 (CIBA-GEIGY)<br>* Ansprüche *<br>----- | 1-25 | |
| | | | **RECHERCHIERTE SACHGEBIETE (Int. Cl.4)** |
| | | | C 07 D 277/00<br>C 07 D 263/00<br>C 07 D 285/00<br>C 07 D 249/00 |

Der vorliegende Recherchenbericht wurde für alle Patentansprüche erstellt

| Recherchenort | Abschlußdatum der Recherche | Prüfer |
|---|---|---|
| DEN HAAG | 25-10-1988 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)